(19) Europäisches Patentamt
European Patent Office
Office européen des brevets

(11) Veröffentlichungsnummer: **0 094 350**
**B1**

(12) **EUROPÄISCHE PATENTSCHRIFT**

(45) Veröffentlichungstag der Patentschrift: **28.02.90**

(21) Anmeldenummer: **83810189.7**

(22) Anmeldetag: **04.05.83**

(51) Int. Cl.[5]: **C 07 D 211/16**, C 07 D 211/50, C 07 D 211/74, C 07 D 211/48, C 07 D 211/44, C 07 D 211/58, C 07 D 491/113, C 07 D 471/10, C 07 D 401/12, C 07 D 401/04, C 08 K 5/34 // (C07D491/113, 319:00, 221:00), (C07D471/10, 235:00, 221:00)

# (54) 1-Diorganocarbamoyl-polyalkylpiperidine und ihre Herstellung.

(30) Priorität: **10.05.82 CH 2890/82**

(43) Veröffentlichungstag der Anmeldung: **16.11.83 Patentblatt 83/46**

(45) Bekanntmachung des Hinweises auf die Patenterteilung: **28.02.90 Patentblatt 90/09**

(84) Bennante Vertragsstaaten: **CH DE FR GB IT LI**

(56) Entgegenhaltungen:
**EP-A-0 006 213**
**EP-A-0 022 079**
**EP-A-0 031 304**
**CH-A-367 171**
**DE-A-2 258 752**
**FR-A-2 351 102**
**US-A-4 069 196**
**JOURNAL OF THE CHEMICAL SOCIETY, 1971, Seiten 1653-1658, London, GB; S.S. BERG et al.: "Acylation of 2,2,6,6-Tetramethylpiperidine and 2,2,5,5-Tetramethylpyrrolidine"**
**JOURNAL FÜR PRAKTISCHE CHEMIE, Band 319, Nr. 3, 1977, Seiten 516-521, Leipzig, DD; H.G. WERCHAN et al.: "Zur Chemie des Triacetonamins. I. Synthese von Derivaten der 2,2,6,6-Tetramethyl-4-oxopiperidin-1-carbonsäure und der 2,2,6,6-Tetramethylpiperidin-1-carbonsäure"**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Karrer, Friedrich, Dr.**
**Rebbergstrasse 5**
**CH-4800 Zofingen (CH)**

EP 0 094 350 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft neue Polyalkylpiperidinderivate, die in 1-Stellung durch eine Diorganocarbamoylgruppe substituiert sind, sowie ein Verfahren zu ihrer Herstellung.

Es ist bekannt, dass sterisch gehinderte Polyalkylpiperidinderivate ausgezeichnete Lichtschutzmittel für organische Materialien, insbesondere für organische Polymere sind. Dabei ist es wichtig, dass der Piperidinring in 2- und 6-Stellung alkyliert ist, während der Stickstoff in 1-Stellung unsubstituiert sein kann oder durch verschiedene organische Gruppen substituiert sein kann. Es wurde in der DE-OS-2 258 752 bereits vorgeschlagen, Polyalkylpiperidinderivate zu verwenden, die am Stickstoff des Piperidinringes eine Carbamoylgruppe $-CO-NH_2$ der eine mono- oder disubstituierte Carbamoylgruppe -CO-NHR bzw. $-CO-NR_2$ besitzen, wobei R einen organischen Rest bezeichnet. Solche Verbindungen mit dem Carbamoylrest -CO-NHR lassen sich aus den NH-Piperidinen durch Umsetzung mit Isocyanaten herstellen (siehe J. prakt. Chemie *319* (1977), 516) und eine Reihe solcher Verbindungen wurden in der DE-OS-2 258 752 im einzelnen beschrieben. Verbindungen mit einer disubstituierten Carbamoylgruppe $-CO-NR_2$ am Piperidin-Stickstoff wurden dort jedoch nur allgemein erwähnt und als allgemeine Herstellungsmethode die Umsetzung der entsprechenden NH-Piperidine mit einem Carbamoylchlorid $Cl-CO-NR_2$ oder mit einem Carbaminsäureester $RO-CO-NR_2$ empfohlen. Versucht man, eine dieser beiden Methoden zur Darstellung von 1-Diorganocarbamoyl-polyalkylpiperidinen zu verwenden, so stösst man auf Schwierigkeiten. Bei Raumtemperatur oder geringfügig erhöhter Temperatur findet keine Umsetzung statt.

Bei höheren Temperaturen erhält man dunkel gefärbte Reaktionsprodukte, aus denen man durch die üblichen Reinigungsmethoden die gewünschten Produkte nur in geringer Ausbeute erhält. Da sich die so erhaltenen N-disubstituierten 1-Carbamoylpiperidine jedoch als Stabilisatoren den monosubstituierten Analogen überlegen erwiesen, war es von erheblichem Interesse, ein technisch brauchbares Herstellungsverfahren für diese Verbindungen zu finden.

Ferner beschrieben S.S. Berg und D.F. Cowling (J. Chem. Soc. (C) *1971*, 1653 - 8) die Umsetzung von 2,2,6,6-Tetramethylpiperidin mit Phosgen im Mol-Verhältnis 2 : 1. Dabei entstand jedoch nicht der gewünschte Harnstoff sondern der Piperidinring wurde gespalten unter Bildung eines Gemisches isomerer nicht-cyclischer Isocyanate.

Es wurde gefunden, dass sich die in 1-Stellung unsubstituierten sterisch gehinderten Polyalkylpiperidine überraschenderweise bereits bei niedrigen Temperaturen mit Phosgen gut umsetzen lassen und dass sich die dabei erhaltenen 1-Chlorcarbonyl-Verbindungen ebenso leicht mit sekundären Aminen zu den entsprechenden 1-Diorganocarbamoyl-Verbindungen weiter umsetzen lassen. Die Chlorcarbonyl-Verbindungen können dabei, ohne isoliert zu werden, mit dem sekundären Amin umgesetzt werden, das heisst, man kann beide Reaktionsstufen als Eintopfreaktion durchführen. Die Herstellung von unsymmetrischen tetrasubstituierten Harnstoffen aus einem sekundären Amin A durch stufenweise Umsetzung mit Phosgen und einem sekundären Amin B ist an sich bekannt und es ist auch bekannt, dass man eine solche Umsetzung in einem inerten Lösungsmittel und in Gegenwart von stöchiometrischen Mengen einer HCl-bindenden Base ausführt. Es war jedoch nicht zu erwarten, dass sich diese Reaktion unter so milden Bedingungen auf die sterisch gehinderten 2,2,6,6-Tetraalkylpiperidine anwenden lässt, da bekannt ist, dass eine Acylierung solcher Piperidine mit Carbonsäurechloriden nur bei erhöhter Temperatur und auch dabei nur relativ langsam abläuft.

Durch dieses Verfahren lassen sich daher neue Piperidinderivate mit einer disubstituierten Carbamoylgruppe $-CO-NR_2$ am Piperidin-Stickstoff herstellen.

Die Erfindung betrifft daher Verbindungen der Formel I

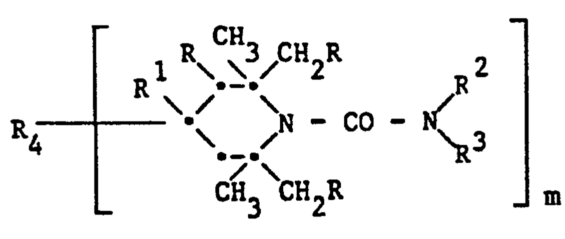

I

worin

m eine ganze Zahl von 1 bis 4 ist,

R Wasserstoff oder $C_1$-$C_4$-Alkyl ist,

$R^1$ Wasserstoff, $C_2$-$C_{12}$-Alkoxy, $C_2$-$C_{20}$-Alkanoyloxy, Benzoyloxy, $C_3$-$C_{25}$-Carbamoyl oder CN ist,

$R^2$ $C_1$-$C_{18}$-Alkyl, $C_3$-$C_{12}$-Alkoxyalkyl, $C_2$-$C_8$-Hydroxyalkyl, $C_3$-$C_{12}$-Alkenyl, $C_7$-$C_{14}$-Aralkyl, $C_6$-$C_{14}$-Aryl, $C_7$-$C_{14}$-Alkaryl, $C_3$-$C_7$-Cycloalkyl oder 2,2,6,6-Tetramethylpiperidin-4-yl ist,

$R^3$ einer der für $R^2$ gegebenen Bedeutungen hat oder

$R^2$ und $R^3$ zusammen mit dem N-Atom, an das sie gebunden sind, einen 5-7-gliedrigen heterocyclischen Ring bilden und

$R^4$ Wasserstoff oder einen m-wertigen organischen Rest bedeutet oder $R^1$ und $R^4$ zusammen einen Oxo-Sauerstoff oder einen zweiwertigen organischen Rest bedeuten.

Ist in Formel I oder Ia R ein Alkylrest, so kann dies z. B. Methyl, Ethyl, Propyl oder Butyl sein. Bevorzugt ist R Wasserstoff.

$R^1$ als Alkoxyrest kann z. B. Methoxy, Ethoxy, Isopropoxy, Butoxy, Hexyloxy, Octyloxy oder Dodecyloxy sein. Bevorzugte Alkoxyreste sind $C_1$-$C_4$-Alkoxyreste.

$R^1$ als Alkanoyloxy kann z. B. Acetoxy, Propionoxy, Hexanoyloxy oder Stearoyloxy sein. $R^1$ als Carbamoyloxy kann mono- oder disubstituiertes Carbamoyloxy sein, wie z. B. Methylcarbamoyloxy, Phenylcarbamoyloxy, Dimethylcarbamoyloxy, Dibutylcarbamoyloxy oder Di(dodecyl)-carbamoyloxy.

Bevorzugt ist $R^1$ Wasserstoff oder eine freie Valenz.

Beispiele für $R^2$ und $R^3$ als Alkyl sind Methyl, Ethyl, Propyl, Isopropyl, Butyl, sec.Butyl, Isoamyl, Hexyl, n-Octyl, 2-Ethylhexyl, 1,1,3,3-Tetramethylbutyl, Decyl, Undecyl, Dodecyl, Hexadecyl oder Octadecyl. Beispiele für $R^2$ und $R^3$ als Alkoxy- oder Hydroxyalkyl sind 2-Methoxyethyl, 2-Ethoxyethyl, 2-Butoxyethyl, 2-Isopropoxyethyl, 3-Butoxypropyl, 2-Octyloxypropyl, 2-Hydroxyethyl, 2-Hydroxypropyl oder 2-Hydroxybutyl. Beispiele für $R^2$ und $R^3$ als Alkenyl sind Allyl, Methallyl oder 2-Butenyl. $R^2$ und $R^3$ als Aralkyl können z. B. Benzyl, 2-Phenylethyl, 3-Phenylpropyl oder 1,1-Dimethylbenzyl sein. $R^2$ und $R^3$ als Aryl oder Alkaryl können z. B. Phenyl, Naphthyl, Tolyl, Xylyl, 4-tert.Butylphenyl oder 4-Octylphenyl sein. $R^2$ und $R^3$ als Cycloalkyl können z. B. Cyclopropyl, Cyclopentyl, Cyclohexyl oder Methylcyclohexyl sein. Wenn $R^2$ und $R^3$ zusammen mit dem N-Atom, an das sie gebunden sind, einen heterocyclischen Ring bilden, so kann dies z. B. ein Piperidin-, Pyrrolidin-, Morpholin-, Piperidin- oder 4-Alkylpiperidinring sein.

Folgende Klassen von erfindungsgemäss herstellbaren Verbindungen sind von besonderer Bedeutung.

a) Verbindungen der Formel III,

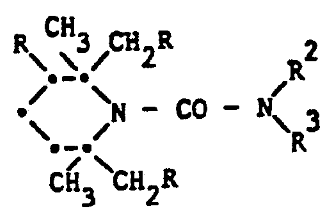

III

worin R, $R^2$ und $R^3$ die oben gegebene Bedeutung haben.

b) Verbindungen der Formel IV,

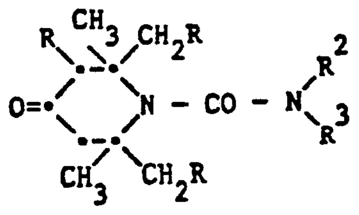

IV

worin R, $R^2$ und $R^3$ die vorhin gegebene Bedeutung haben.

c) Verbindungen der Formel V,

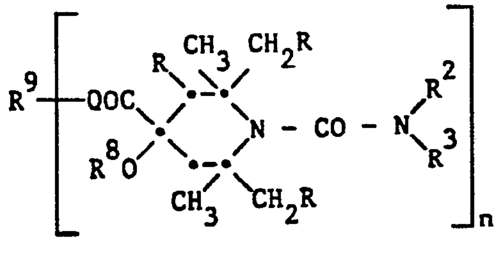

V

worin n 1 bis 4 ist,
$R^8$ $C_1$-$C_4$-Alkyl oder $C_2$-$C_{12}$-Alkanoyl ist, $R^9$ der n-wertige Rest eines $C_1$-$C_{20}$-Alkohols, $C_2$-$C_{16}$-Diols, $C_3$-$C_{18}$-Triols oder $C_4$-$C_{20}$-Tetrols ist, der durch ein oder mehrere Sauerstoffatome unterbrochen sein kann, und R, $R^2$ und $R^3$ die oben

gegebene Bedeutung haben.

Beispiele für $R^9$ als Alkoholreste sind die einwertigen Reste von Methanol, Ethanol, Isopropanol, tert.Butanol, Isopentanol, 2-Methoxyethanol, n-Hexanol, Cyclohexanol, 2-Ethylhexanol, Isooctanol, Cyclooctanol, n-Decanol, Benzylalkohol, n-Dodecanol oder n-Octadecanol; die zweiwertigen Reste von Ethylenglykol, Propandiol-1,2, Butandiol-1,4, Hexandiol-1,6, 2,2,4-Trimethylhexandiol-1,6, Dodecandiol-1,12, Xylylenglykol, 1,4-Di(hydroxymethyl)cyclohexan, Diethylenglykol oder Triethylenglykol; die dreiwertigen Reste von Glycerin, Trimethylolethan oder Trimethylolpropan und der vierwertige Rest von Pentaerythrit, die durch Abspaltung von n Hydroxylgruppen aus den n-wertigen Alkoholen entstehen.

d) Verbindungen der Formel VI,

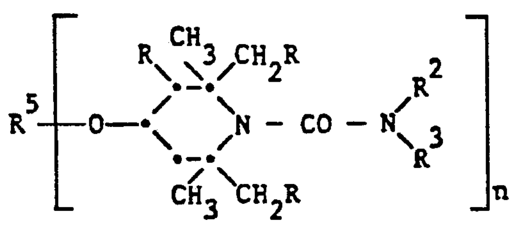

VI

worin n eine ganze Zahl von 1 bis 4 ist,

$R^5$, wenn n = 1 ist, Wasserstoff, wenn n = 2 ist, $C_2$-$C_{12}$-Alkylen, $C_4$-$C_{12}$-Alkenylen, Xylylen, einen zweiwertigen Acylrest einer aliphatischen, cycloaliphatischen, araliphatischen, aromatischen oder heterocyclischen Dicarbonsäure, Dicarbaminsäure oder Phosphor enthaltenden Säure, wenn n = 3 ist, einen dreiwertigen Acylrest einer aliphatischen, cycloaliphatischen, araliphatischen, aromatischen oder heterocyclischen Tricarbonsäure, Tricarbaminsäure oder Phosphor enthaltenden Säure, wenn n = 4 ist, einen vierwertigen Acylrest einer aliphatischen, cycloaliphatischen oder aromatischen Tetracarbonsäure bedeutet und

R, $R^2$ und $R^3$ die oben gegebene Bedeutung haben.

$R^5$ als zweiwertiger Acylrest kann z. B. der Rest von Oxal-, Malon-, Bernstein-, Glutar-, Diethylmalon-, Dodecylbernstein-, Dibenzylmalon-, Adipin-, Sebacin-, Malein-, Fumar-, Diglykol-, Isophthal-, Terephthal-, Diphenyl-4,4'-dicarbon-, Tetrahydrophthal-, Hexahydroterephthal-, Decahydronaphthalin-1,4-dicarbon-, Hexamethylendicarbamin-, Toluylen-2,4-dicarbamin- oder Phenylphosphonsäure sein.

Beispiele für $R^5$ als drei- und vierwertige Acylreste sind die Reste von Tricarballyl-, Trimellith-, Nitrilotriessig-, Phosphor-, Phosphorig-, Pyromellith-, Cyclohexanon-2,2,6,6-tetracarbon- oder 4,4'-Methylendiphthalsäure.

$R^5$ kann auch einen Acylrest einer Di- oder Tricarbonsäure bedeuten, wie sie technisch durch Di- oder Trimerisierung von ungesättigten Fettsäuren, beispielsweise von Linolsäure oder durch Diels-Alder-Addition von Acrylsäure an Linolsäure hergestellt werden.

e) Verbindungen der Formel VII,

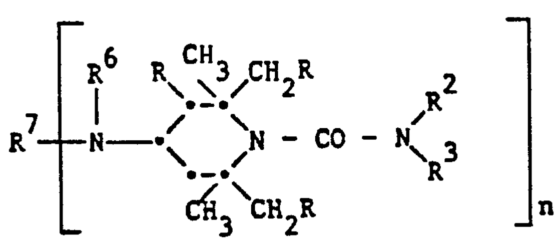

VII

worin n 1 oder 2 ist,

$R^6$ $C_1$-$C_{12}$-Alkyl, $C_3$-$C_{12}$-Alkoxyalkyl, $C_5$-$C_8$-Cycloalkyl, $C_7$-$C_9$-Aralkyl, $C_2$-$C_{18}$-Alkanoyl, $C_3$-$C_5$-Alkenoyl, Benzoyl, $C_2$-$C_{13}$-Alkoxycarbonyl oder $C_7$-$C_{11}$-Aryloxycarbonyl bedeutet, und

$R^7$, wenn n = 1 ist, $C_1$-$C_{12}$-Alkyl, $C_3$-$C_{12}$-Alkoxyalkyl, $C_5$-$C_8$-Cycloalkyl, $C_3$-$C_8$-Alkenyl, Glycidyl oder Cyanethyl ist, und wenn n = 2 ist, $C_2$-$C_{12}$-Alkylen, $C_6$-$C_{15}$-Arylen, Xylylen eine Gruppe $-CH_2-CH(OH)-CH_2-$ oder $-CH_2-CH(OH)-CH_2-O-D-O-CH_2-CH(OH)-CH_2-$ bedeutet, worin D $C_2$-$C_{10}$-Alkylen, $C_6$-$C_{15}$-Arylen oder $C_6$-$C_{12}$-Cycloalkylen ist, oder im Falle, dass $R^6$ Alkyl, Cycloalkyl oder Aralkyl ist,

$R^7$ auch ein zweiwertiger Acylrest einer aliphatischen, cycloaliphatischen oder aromatischen Dicarbonsäure oder Dicarbaminsäure sein kann, oder $R^6$ und $R^7$ zusammen mit dem N-Atom im Falle von n = 1 ein Imidrest einer aliphatischen, cycloaliphatischen oder aromatischen 1,2-Dicarbonsäure mit 4 bis 12 C-Atomen sind.

$R^6$ und $R^7$ als Alkyl können beispielsweise Methyl, Ethyl, Isopropyl, n-Butyl, tert.Butyl, Isoamyl, n-Hexyl, n-Octyl, 2-Ethylhexyl, n-Decyl oder n-Dodecyl sein. $R^7$ als Alkenyl kann z. B. Allyl, Methallyl, 2-Butenyl-1 oder 1-Dimethylallyl sein. $R^6$ und $R^7$ als Cycloalkyl können z. B. Cyclopentyl, Cyclohexyl, Cycloheptyl oder Cyclooctyl sein.

$R^6$ als Aralkyl kann z. B. Benzyl, Phenylethyl oder Phenylpropyl sein. $R^6$ als Acylrest kann z. B. Acetyl, Propionyl, Butyroyl, Hexanoyl, Octanoyl, Lauroyl, Palmitoyl, Stearoyl, Acryloyl, Methacryloyl, Benzoyl, Ethoxycarbonyl, Butoxycarbonyl, Dodecyloxycarbonyl, Phenoxycarbonyl oder Tolyloxycarbonyl sein.

$R^7$ als Alkylen kann z. B. 1,2-Ethylen, 1,3-Propylen, Tetra-, Hexa-, Octa- oder Dodecamethylen sein. $R^7$ als Arylen kann z. B. Phenylen, Tolylen, Diphenylen, Diphenylenmethan oder Diphenylen-2,2-propan sein. $R^7$ als zweiwertiger Acylrest kann z. B. Oxalyl, Succinoyl, Adipoyl, Sebacoyl, Cyclohexandicarbonyl, Terephthaloyl oder Hexamethylendicarbamoyl sein.

Im Falle von n = 1 kann $R^6$ und $R^7$ zusammen mit dem N-Atom ein cyclischer Imidrest sein, wie z. B. ein Succinimid-, Maleinimid-, Phthalimid- oder Hexahydrophthalimidrest sein.

f) Verbindungen der Formel VIII,

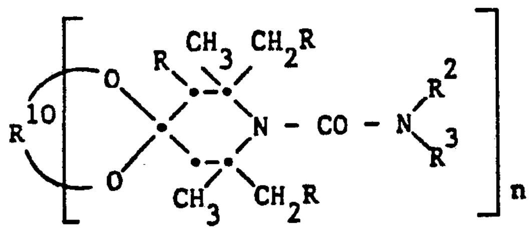

VIII

worin n 1 oder 2 ist,
$R^{10}$ im Falle von n = 1 $C_2$-$C_8$-Alkylen oder $C_4$-$C_{22}$-Acyloxyalkylen und im Falle von n = 2 die Gruppe $(-CH_2)_2C(CH_2-)_2$ bedeutet und R, $R^2$ und $R^3$ die oben gegebene Bedeutung haben.

$R^{10}$ kann darin z. B. 1,2-Ethylen, 1,2-Propylen, 1,3-Propylen, 2,2-Dimethylpropylen-1,3, 1,2-Octylen, 2-(Acetoxymethyl)-2-ethyl-propylen-1,3, 2-(Lauroyloxymethyl)-2-ethyl-propylen-1,3, 2-(Butyroyloxymethyl)-2-methyl-propylen-1,3 oder 2-Acetoxy-propylen-1,3 sein.

g) Verbindungen der Formel IX,

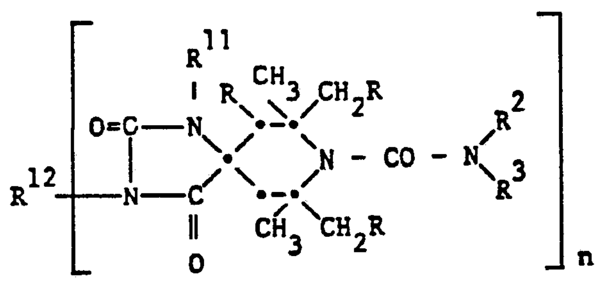

IX

worin n 1 oder 2 ist,
$R^{11}$ Wasserstoff, $C_1$-$C_{12}$-Alkyl, Allyl, Benzyl, Glycidyl oder $C_2$-$C_6$-Alkoxyalkyl ist, $R^{12}$ bei n = 1 Wasserstoff, $C_1$-$C_{12}$-Alkyl, $C_3$-$C_5$-Alkenyl, $C_7$-$C_9$-Aralkyl, $C_5$-$C_8$-Cycloalkyl, $C_2$-$C_4$-Hydroxyalkyl, $C_3$-$C_6$-Alkoxyalkyl, $C_6$-$C_{10}$-Aryl oder Glycidyl und bei n = 2 $C_2$-$C_{12}$-Alkylen, $C_6$-$C_{15}$-Arylen oder $C_4$-$C_8$-Alkenylen oder eine Gruppe $-CH_2-CH(OH)-CH_2-O-D-O-CH_2-CH(OH)-CH_2-$ bedeutet, worin

D $C_2$-$C_{12}$-Alkylen, $C_6$-$C_{15}$-Arylen oder $C_6$-$C_{12}$-Cycloalkylen ist, und R, $R^3$ und $R^3$ die oben angegebene Bedeutung haben.

$R^{11}$ und $R^{12}$ können darin unverzweigtes oder verzweigtes Alkyl sein, wie z. B. Methyl, Ethyl, Isopropyl, n-Butyl, sec.Butyl, tert.Butyl, n-Hexyl, n-Octyl, 2-Ethylhexyl, n-Decyl oder n-Dodecyl. $R^{12}$ als Alkenyl kann z. B. Allyl, Methallyl oder 1,1-Dimethylallyl sein. $R^{11}$ als Alkoxyalkyl kann zu. B. 2-Methoxyethyl, 2-Butoxyethyl oder 3-Ethoxypropyl sein.

$R^{12}$ als Hydroxy- oder Alkoxyalkyl kann z. B. 2-Hydroxyethyl, 2-Hydroxypropyl oder 2-Ethoxyethyl sein.

$R^{12}$ als Aralkyl kann z. B. Benzyl, 2-Phenylethyl oder 1,1-Dimethylbenzyl sein. $R^{12}$ als Cycloalkyl kann z. B. Cyclopentyl, Cyclohexyl, Cycloheptyl oder Cyclooctyl sein.

$R^{12}$ als Alkylen kann z. B. 1,2-Ethylen, 1,3-Propylen, Tetra-, Hexa-, Octa-, Deca- oder Dodecamethylen sein. K als Alkenylen kann z. B. 2-Butenylen-1,4 oder 3-Hexenylen-1,6 sein.

$R^{12}$ als Arylen kann z. B. Phenylen, Naphthylen, Diphenylen oder 2,2-Diphenylenpropan sein.

h) Verbindungen der Formel XII,

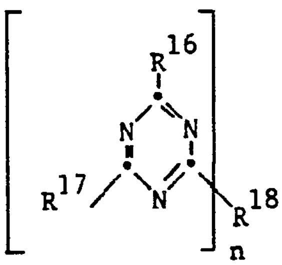

XII

worin n 1 oder 2 ist,
$R^{16}$ eine Gruppe der Formel

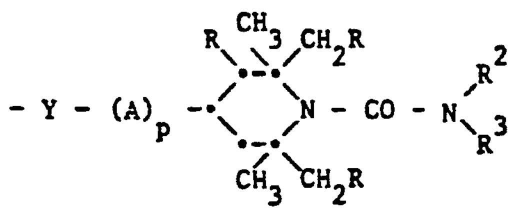

ist, worin R, $R^2$ und $R^3$ die in Anspruch 1 gegebene Bedeutung haben, Y -O- oder $-NR^{19}-$ ist, $R^{19}$ H, $C_1$,-$C_{12}$-Alkyl, $C^2$-$C^4$-Hydroxyalkyl, $C^3$-$C^6$-Alkoxyalkyl, Cyclohexyl, Benzyl oder eine Gruppe

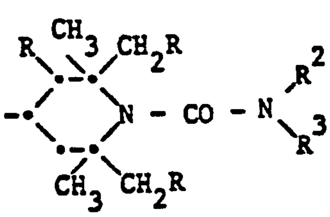

darstellt, A $C_2$-$C_6$-Alkylen oder $-(CH_2)_3-O-$ und p Null oder 1 bedeuten,

$R^{17}$ eine der für $R^{16}$ gegebenen Bedeutungen hat oder $-NR^{20}R^{21}$, $-OR^{22}$, $-NHCH_2OR^{23}$ oder $-N(CH_2OR^{23})_2$ bedeutet, worin $R^{20}$ eine der für $R^{19}$ gegebenen Bedeutungen hat, $R^{21}$ $C_1$-$C_{12}$-Alkyl, Cyclohexyl oder Benzyl ist oder $R^{20}$ und $R^{21}$ zusammen $C_4$-$C_5$-Alkylen oder Oxaalkylen sind,

$R^{22}$ Wasserstoff, $C_1$-$C_{12}$-Alkyl oder Phenyl ist und $R^{23}$ Wasserstoff oder $C_1$-$C_4$-Alkyl bedeutet, und $R^{18}$ bei n = 1 eine der für $R^{16}$ und $R^{17}$ gegebenen Bedeutungen hat und bei n = 2 eine Gruppe -Y-Q-Y- darstellt, worin Q $C_2$-$C_{12}$-Alkylen, durch -O-, -NH-, -N-Alkyl oder durch eine Gruppe der Formel

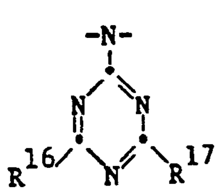

unterbrochenes $C_4$-$C_{12}$-Alkylen, Cyclohexylen, Xylylen oder Phenylen bedeutet.

Darin können $R^{19}$, $R^{21}$ und $R^{22}$ Alkyl sein und können unverzweigtes oder verzweigtes Alkyl sein, wie z. B. Methyl, Ethyl, Isopropyl, tert.Butyl, Isoamyl, n-Hexyl, 2-Ethylhexyl, Isononyl, n-Decyl oder n-Dodecyl. $R^{19}$ als Alkoxyalkyl kann z. B. 2-Methoxyethyl, 2-Butoxyethyl oder 2-Ethoxybutyl sein.

A und Q als Alkylen können unverzweigt oder verzweigt sein, wie z. B. 1,2-Ethylen, 1,3-Propylen, 1,2-Butylen oder 1,2-Hexylen. Q kann darüberhinaus auch z. B. Hexa-, Octa-, Deca-, Dodeca- oder 2,4,4-Trimethylhexamethylen sein. Q als unterbrochenes Alkylen kann z. B. 3-Oxapentylen-1,5, 3,6-Dioxaoctylen-1,8, 3-Azapentylen-1,5 oder 3-(Methylaza)-pentylen-1,5 sein.

$R^{20}$ und $R^{21}$ können zusammen $C_4$-$C_5$-Alkylen oder Oxaalkylen sein.
In diesem Fall bilden sie zusammen mit dem N-Atom, an das sie gebunden sind, einen gesättigten heterocyclischen Ring, wie z. B. einen Pyrrolidin-, Piperidin- oder Morpholinring.

i) Verbindungen der Formel XIII,

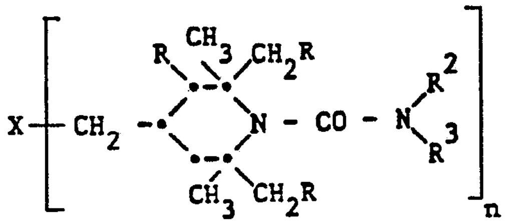

XIII

worin n 1 oder 2 ist,
X, wenn n = 1 ist, -CN, $-COOR^{24}$, $-CH_2NH_2$, $-CH_2OH$ oder $-CHCOOR^{24}$ bedeutet, worin $R^{24}$ $C_1$-$C_{18}$-Alkyl, Benzyl oder Cyclohexyl bedeutet, und wenn n = 2 ist, $-COO-R^{25}-OOC-$ bedeutet, worin $R_{25}$ $C_2$-$C_{12}$-Alkylen, durch -O- oder -N($C_1$-$C_4$-Alkyl)- unterbrochenes $C_4$-$C_{10}$-Alkylen, $C_6$-$C_{15}$-Cycloalkylen, p-Xylylen oder Hexahydroxylylen bedeutet und R, $R^2$ und $R^3$ die vorhin gegebenen Bedeutungen haben.

$R^{24}$ kann darin ein unverzweigter oder verzweigter Alkylrest sein wie z. B. Methyl, Butyl, n-Octyl, tert.Octyl, 2-Ethyloxyl, n-Dodecyl oder Octadecyl. $R^{25}$ als Alkylen oder unterbrochenes Alkylen kann z. B. 1,2-Ethylen, 1,2-Propylen, Tetramethylen, Hexamethylen, Octamethylen, 2,4,4-Trimethylhexamethylen, 2,2-Dimethylpropylen-1,3, 3-Oxapentylen-1,5, 3,6-Dioxaoctylen-1,8 oder 3-(Methylaza)-pentylen-1,5 sein. Beispiele für $R^{25}$ als Cycloalkylen sind 1,4-Cyclohexylen, 4,4'-Dicyclohexylen, 2,2-Di(cyclohexylen)-propan oder Decahydronaphthylen-1,4.

Unter allen Verbindungen der Klassen a) bis i) sind jeweils solche Verbindungen bevorzugt, die eine Gruppe der Formel I enthalten, worin R Wasserstoff ist.

Die Erfindung betrifft ferner ein Verfahren zur Herstellung von Verbindungen der Formel I, wie vorhin definiert, durch Umsetzung von Verbindungen der Formel II

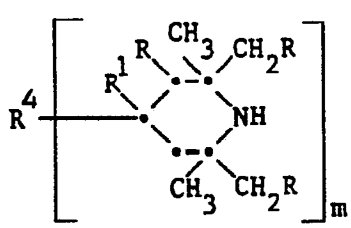

II

mit Phosgen in einem inerten Lösungsmittel in Gegenwart von molaren Mengen einer Base und anschliessende Umsetzung mit einem sekundären Amin der Formel $R^2$-NH-$R^3$ ebenfalls in Gegenwart von molaren Mengen einer Base, wobei als Base in der ersten Stufe auch ein Überschuß der Verbindung II und in der zweiten Stufe auch ein Überschuß der Verbindung $R^2$-NH-$R^6$ verwendet werden kann.

Das erfindungsgemässe Verfahren umfasst zwei Reaktionsstufen. In der ersten Stufe wird die Verbindung der Formel II in eine Verbindung der Formel IIa übergeführt, die in der zweiten Stufe in die Verbindung der Formel I verwandelt wird:

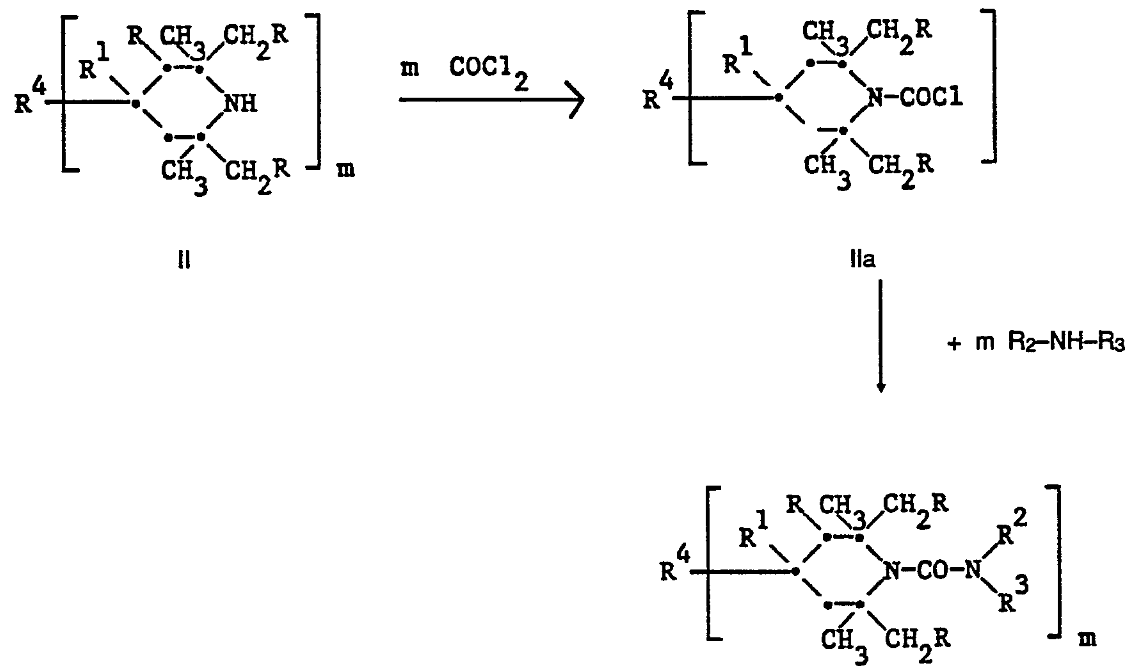


II

IIa

(I)

Wie bereits erwähnt, können beide Reaktionsstufen als Eintopfverfahren ausgeführt werden, das heisst, das Zwischenprodukt der Struktur IIa braucht nicht isoliert zu werden.

Beide Stufen werden in einem inerten Lösungsmittel ausgeführt. Als solches eignet sich ein Kohlenwasserstoff wie z. B. Benzol, Toluol, Xylol, Cyclohexan oder ein Alkan-Gemisch; ein Ester wie z. B. Methylacetat, Ethylacetat oder Butylacetat; oder ein chloriertes Lösungsmittel wie z. B. Methylenchlorid, Ethylenchlorid oder Tetrachlormethan. Besonders vorteilhaft sind solche Lösungsmittel, in denen alle Edukte und die N-Carbamoylverbindung gut löslich, jedoch das Basen-Hydrochlorid unlöslich sind. In diesem Fall kann das Hydrochlorid in einfacher Weise durch Filtration abgetrennt werden.

Als Base kann jeder Protonenakzeptor verwendet werden, insbesondere sind organische Amine geeignet. Als Base kann auch ein Überschuß der Edukte verwendet werden, also in der ersten Stufe ein Überschuß der Verbindung mit der Gruppe Ia, in der zweiten Stufe ein Überschuss der Verbindung $R^2$-NH-$R^3$. Bevorzugt setzt man jedoch ein tertiäres Amin als Hilfsbase zu, wie z. B. ein Trialkylamin, ein Dialkylanilin oder eine heterocyclische Base.

Für jede der beiden Reaktionsstufen wird je 1 Moläquivalent Base benötigt. Wird in der ersten Stufe ein Überschuß des Eduktes mit der Gruppe der Formel II verwendet, so gibt man pro Moläquivalent NH etwa 0,5 Moläquivalente Phosgen zu. Verwendet man jedoch eine Hilfsbase, so gibt man pro Moläquivalent NH etwa 1 Mol Phosgen und mindestens 1 Mol Hilfsbase zu. In entsprechender Weise werden in der zweiten Reaktionsstufe pro Moläquivalent -COCl entweder mindestens 2 Moläquivalente $R^2NHR^3$ zugegeben oder 1 Moläquivalent $R^2NHR^3$ und mindestens 1 Moläquivalent der Hilfsbase. Ein kleiner Überschuss an Protonenakzeptor ist im allgemeinen von Vorteil.

Vorzugsweise verwendet man pro Mol II 0,9 - 1,2 Mol Phosgen, mindestens ein Mol des sekundären Amins und mindestens 2 Mol einer Base.

Beide Reaktionsstufen können bei Raumtemperatur oder gering erhöhter oder erniedrigter Temperatur durchgeführt werden, vorzugsweise bei Temperaturen unterhalb 40°C. Die erste Reaktionsstufe lässt sich bereits bei überraschend tiefen Temperaturen wie z. B. bei -30 bis +20°C, ausführen.

Das gebildete Basen-Hydrochlorid kann nach der ersten Reaktionsstufe abfiltriert werden. Einfacher ist es jedoch die zweite Reaktionsstufe ohne Zwischen-Filtration unmittelbar anzuschliessen und erst nach der zweiten Reaktionsstufe zu filtrieren. Es ist für den Fachmann selbstverständlich, dass beide Reaktionsstufen unter Ausschluss von Feuchtigkeit geschehen müssen, dasselbe gilt für eine eventuelle Zwischenfiltration.

Aus dem Filtrat der zweiten Reaktionsstufe wird das Produkt durch Abdestillieren des Lösungsmittels als Destillierrückstand isoliert und kann gegebenenfalls durch Umkristallisieren oder eine andere übliche Methode gereinigt werden.

Die so erhaltenen 1-Diorganocarbamoylpiperidine sind in reiner Form sehr stabile Verbindungen und eignen sich als Stabilisatoren für organische Materialien, insbesondere gegen deren Schädigung durch Lichteinwirkung. Solche gegen Licht zu schützende Materialien können z. B. Öle, Fette, Wachse, Detergentien oder Lösungsmittel sein, insbesondere aber eignen sich die erfindungsgemäss herstellbaren Verbindungen als Stabilisatoren für organische Polymere. Beispiele von Polymeren, die gegen Lichteinwirkung empfindlich sind und die durch den Zusatz

der erfindungsgemäss herstellbaren Verbindungen stabilisiert werden können, sind die folgenden Klassen von Polymeren.

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen, wie z. B. von Cyclopenten oder Norbornen.
2. Mischungen der unter 1) genannten Polymeren, z. B. Mischungen von Polypropylen mit Polyäthylen oder mit Polyisobutylen.
3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Isomere), sowie Terpolymere von Ethylen mit Propylen und einem Dien, wie Hexadien, Dicyclopentadien oder Ethylidennorbornen.
4. Polystyrol.
5. Copolymere von Styrol oder α-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methylacrylat; Mischungen von hoher Schlagzähigkeit aus Styrol-Copolymeren und einem anderen Polymer, wie z. B. einem Polyacrylat, einem Dien-Polymeren oder einem Ethylen-Propylen-Dien-Terpolymeren; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie z. B. als sogenannte ABS, MBS, ASA oder AES-Polymere bekannt sind.
7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere, Methylmethacrylat-Styrol-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyrat, Polyallylphthalat, Polyallylmelamin.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z. B. Ethylenoxyd enthalten.
13. Polyphenylenoxyde und -sulfide und deren Mischungen mit Styrolpolymeren.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte (Polyisocyanate, Polyole, Präpolymere).
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Copolymere mit Polyethern, wie z. B. mit Polyethylenglykol, Polypropylenglykol oder Polytetramethylenglykol.
16. Polyharnstoffe, Polyimide, Polyamid-imide und Polybenzimidazole.
17. Polyester, die sich von Dicarbonsäuren und Diolen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-[-2,2-bis(4-hydroxyphenyl)-propan-]-terephthalat, Polyhydroxybenzoate, sowie Block-Polyether-ester, die sich von Polyethern mit Hydroxyendgruppen, Dialkoholen und Dicarbonsäuren ableiten.
18. Polycarbonate.
19. Polysulfone und Polyethersulfone.
20. Vernetzte Polymere, die sich von Aldehyden einerseits und Phenolen, Harnstoff und Melamin andererseits ableiten, wie Phenol-Formaldehyd-, Harnstoff-Formaldehyd- und Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten, wie z. B. von Epoxyacrylaten, Urethan-acrylaten oder Polyesteracrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z. B. von Bis-glycidylethern oder von cycloaliphatischen Diepoxiden.
26. Natürliche Polymere, wie Cellulose, Naturkautschuk, Gelatine, sowie deren polymeranalog chemisch abgewandelten Derivate, wie Celluloseacetate, -propionate und -butyrate, bzw. die Celluloseether, wie Methylcellulose.

Die Stabilisatoren werden den Kunststoffen in einer Konzentration von 0,01 bis 5 Gew.-%, berechnet auf das zu stabilisierende Material, zugesetzt. Vorzugsweise werden 0,03 bis 1,5, besonders bevorzugt 0,2 bis 0,6 Gew.-% der Verbindungen, berechnet auf das zu stabilisierende Material, in dieses eingearbeitet.

Die Einarbeitung kann während oder nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Ausser den Verbindungen der Formel I können den Kunststoffen auch noch andere, bekannte Stabilisatoren zugesetzt werden. Dies können z. B. Antioxydantien, Lichtschutzmittel oder Metalldesaktivatoren sein, oder auch Costabilisatoren, wie z. B. solche vom Typ der Phosphorigsäureester. Weiterhin können sonstige in der Kunststofftechnologie übliche Zusätze wie z. B. Flammschutzmittel, Antistatika, Weichmacher, Gleitmittel, Treibmittel, Pigmente, Verstärkungsstoffe oder Füllstoffe zugesetzt werden. Einzelne Beispiele solcher bekannter und üblicher Zusätze sind die folgenden Verbindungen:

**1. Antioxidantien**

**1.1. Alkylierte Monophenole** wie 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-di-methylphenol, 2,6-Di-octadecyl-4-methylphenol, 2,4,6-Tri-cyclohexylphenol, 2,6-Di-tert.butyl-4-methoxymethylphenol.
**1.2. Alkylierte Hydrochinone**, wie 2,6-Di-tert.butyl-4-methoxyphenol, 2,5-Di-tert.butyl-hydrochinon, 2,5-Di-tert.amyl-hydrochinon, 2,6-Di-phenyl-4-octadecyloxyphenol.
**1.3. Hydroxylierte Thiodiphenylether** wie 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).
**1.4. Alkyliden-Bisphenole** wie 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan,
Ethylenglycol-bis-[3,3-bis-(3'-tert.butyl-4'-hydroxyphenyl)-butyrat], Di-(3-tert.butyl-4-hydroxy-5-methylphenyl)-dicyclopentadien, Di-[2-(3'-tert.butyl-2'-hydroxy-5'-methyl-benzyl)-6-tert.butyl-4-methyl-phenyl]-terephthalat.
**1.5. Benzylverbindungen** wie 1,3,5-Tri-(3,5-di-tert.butyl-4-hydroxybenzyl)-2,4,6-trimethylbenzol, Di-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)-dithiolterephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, 3,5-Di-tert.butyl-4-hydroxybenzyl-phosphonsäure-monoethylester-Calcium-salz.
**1.6. Acylaminophenole**, wie 4-Hydroxy-laurinsäureanilid, 4-Hydroxy-stearinsäureanilid, 2,4-Bis-octylmercapto-6-(3,5-di-tert.butyl-4-hydroxyanilino)-s-triazin.
**1.7. Ester der β-(3,5-Di-tert.butyl-4-hydroxyphenyl)-propionsäure**
mit ein- oder mehrwertigen Alkoholen wie z. B. mit Methanol, Octadecanol, 1,6-Hexandiol, Neopentylglycol, Thiodiethylenglykol, Diethylenglycol, Triethylenglycol, Pentaerythrit, Tris-hydroxyethylisocyanurat, Di-hydroxyethyl-oxalsäurediamid.
**1.8. Ester der β-(5-tert.Butyl-4 hydroxy-3-methylphenyl)-propionsäure**
mit ein- oder mehrwertigen Alkoholen, wie z. B. mit Methanol, Octadecanol, 1,6-Hexandiol, Neopentylglycol, Thiodiethylenglycol, Diethylenglycol, Triethylenglycol, Pentaerythrit, Tris-hydroxyethylisocyanurat, Di-hydroxyethyl-oxalsäurediamid.
**1.9. Amide der β-(3,5-Di-tert.butyl-4-hydroxyphenyl)-propionsäure,**
wie z. B. N,N'-Di-(3,5-di-tert.butyl-4-hydroxyphenylpropionyl)-hexamethylendiamin, N,N'-Di-(3,5-di-tert.butyl-4-hydroxyphenylpropionyl)-trimethylendiamin, N,N'-Di-(3,5-di-tert.butyl-4-hydroxyphenylpropionyl)-hydrazin.

**2. UV-Absorber und Lichtschutzmittel**

**2.1. 2-(2'-Hydroxyphenyl)-benztriazole**, wie z. B. das 5'-Methyl-, 3',5'-Di-tert.butyl-, 5'-Tert.butyl-, 5'-(1,1,1,3,3-Tetrame-

thylbutyl)-, 5-Chlor-3',5'-di-tert.butyl-, 5-Chlor-3'-tert.butyl-5'-methyl-, 3'-sec.Butyl-5'-tert.butyl-, 4'-Octoxy-, 3',5'-Di-tert.amyl-Derivat.

**2.2. 2-Hydroxybenzophenone**, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

**2.3. Ester von gegebenenfalls substituierten Benzoesäuren**, wie z. B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butyl-phenylester.

**2.4. Acrylate**, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

**2.5. Nickelverbindungen**, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triäthanolamin oder N-Cyclohexyl-diethanolamin, Nickeldibutyldi-thiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxypyrazols, gegebenenfalls mit zusätzlichen Liganden.

**2.6. Sterisch gehinderte Amine**, wie z. B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin. Tris-(2,2,6,6-tetramethyl-4-piperidyl)-nitriloacetat.

**2.7. Oxalsäurediamide**, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-di-methylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho- und para-Methoxy- sowie von o- und p-Ethoxy-disubstituierten Oxaniliden.

**3. Metalldesaktivatoren**, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

**4. Phosphite und Phosphonite**, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythrit-diphosphit, Tris-(2,4-di-tert.butylphenyl)-phosphit, Diisodecyl-pentaerythrit-diphosphit, Di-(2,4-di-tert.butylphenyl)-pentaerythrit-diphosphit, Tristearyl-sorbit-triphosphit, Tetrakis-(2,4-di-tert.butylphenyl)-4,4'-biphenylen-diphosphonit.

**5. Peroxidzerstörende Verbindungen**, wie z. B. Ester der β-Thio-di-propionsäure, beispielsweise der Lauryl-, Stearyl-, Myristyl- oder Tridecylester, Mercaptobenzimidazol, das Zinksalz des 2-Mercapto-benzimidazols, Zink-dibutyl-dithiocarbamat, Dioctadecyldisulfid, Pentaerythrit-tetrakis-(β-dodecylmercapto)-propionat.

**6. Polyamidstabilisatoren**, wie z. B. Kupfersalze in Kombination mit Jodiden und/oder Phosphorverbindungen und Salze des zweiwertigen Mangans.

**7. Basische Co-Stabilisatoren**, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

**8. Nukleierungsmittel**, wie z. B. 4-tert.Butylbenzoesäure, Adipinsäure, Diphenylessigsäure.

**9. Füllstoffe und Verstärkungsmittel**, wie z. B. Calciumcarbonate, Silikate, Glasfasern, Asbest, Talk, Kaolin, Glimmer, Bariumsulfat, Metalloxide und -hydroxide, Russ, Graphit.

**10. Sonstige Zusätze**, wie z. B. Weichmacher, Gleitmittel, Emulgatoren, Pigmente, Optische Aufheller, Flammschutzmittel, Antistatika, Treibmittel.

Die Erfindung betrifft daher auch die durch Zusatz von 0,01 bis 5 Gew.-% einer erfindungsgemäss hergestellten Verbindung stabilisierten organischen Polymere, die gegebenenfalls noch andere bekannte und übliche Zusätze enthalten können. Die so stabilisierten Kunststoffe können in verschiedenster Form angewendet werden, z. B. als Folien, Fasern, Bändchen, Profile oder als Bindemittel für Lacke, Klebemittel oder Kitte.

Die erfindungsgemäss hergestellten Verbindungen können weiterhin als Zwischenprodukt für die Herstellung anderer Polyalkylpiperidinderivate, die ebenfalls Lichtschutzwirkung besitzen, verwendet werden. Durch Reaktionen, die die 1-Diorganocarbamoylgruppe nicht verändern, können Verbindungen hergestellt werden, die sich durch direkte Phosgenierung nicht herstellen lassen.

Beispielsweise lässt sich eine Verbindung der Formel XIV durch Hydrolyse in ein 1-Carbamoyl-4-hydroxypiperidin XV überführen, welches durch direkte Phosgenierung von 4-Hydroxy-tetramethylpiperidin nicht gut zugänglich ist:

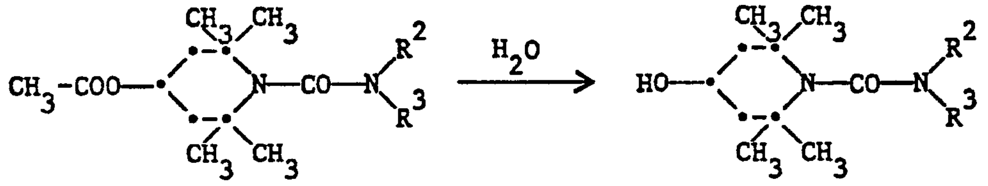


XIV XV

Alternativ kann XV auch durch Reduktion der entsprechenden 4-Oxo-verbindung XVI hergestellt werden:

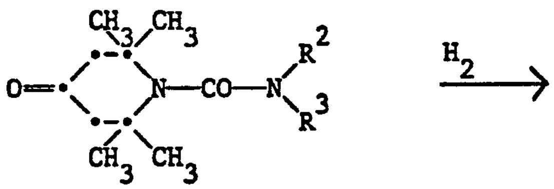


Hierzu eignet sich eine Reduktion mit komplexen Borhydriden oder eine katalytische Hydrierung.

Die 4-Hydroxyverbindung XV lässt sich wiederum weiter umsetzen, beispielsweise durch Veretherung, Veresterung oder Carbamoylierung der Hydroxylgruppe.

Die folgenden Beispiele beschreiben das erfindungsgemässe Verfahren und einzelne der damit erhältlichen Verbindungen sowie die Umwandlung solcher Verbindungen durch Folgereaktionen in andere Verbindungen der Formel I. Die darin enthaltenen Temperaturangaben (°) bedeuten °C.

**Beispiel 1**

**Carbamoylierung in Ethylacetat**

Zu einer Lösung von 99,7 g (0,5 Mol) 4-Acetoxy-2,2,6,6-tetramethylpiperidin in 400 ml Essigsäureethylester wird unter Rühren bei 20 - 22° innerhalb von 6 Std. die Lösung von 24,7 g (0,25 Mol) Phosgen in ca. 150 ml Essigsäureethylester getropft. Nach weiteren 2 Std. Rühren bei Raumtemperatur tropft man nun in ca. 30 Min. 63 ml (0,6 Mol) Diethylamin zum Reaktionsgemisch. Nach weiteren 14 Std. Rühren bei Raumtemperatur wird das Reaktionsgemisch filtriert und der Salzrückstand mit Hexan gut nachgewaschen. Die vereinigten Filtrate werden zweimal mit Wasser, dreimal mit eiskalter n-Salzsäure und wieder mit Wasser gewaschen über Natriumsulfat getrocknet und die Lösungsmittel im Vakuumrotationsverdampfer abdestilliert. Das Rohprodukt wird in Diisopropylether umkristallisiert, wodurch reines 1-Diethylcarbamoyl-4-acetoxy-2,2,6,6-tetramethylpiperidin (Verbindung Nr. 1) vom Smp. 58 - 60° erhalten wird.

Elementaranalyse:

| | | | |
|---|---|---|---|
| $C_{16}H_{30}N_2O_3$ | Ber.: C 64,40 | H 10,13 | N 9,39 % |
| (298,4) | Gef.: C 64,6 | H 10,0 | N 9,3 % |

Das $^1$H-NMR-Spektrum steht mit der angegebenen Struktur in Einklang.

Analog Beispiel 1 werden die in Tabellen 1 aufgeführten N-Carbamoylpiperidine hergestellt.

**Tabelle 1**

| Verbindung Nr. | Name | Formel | Physikalische Daten |
|---|---|---|---|
| 2 | 1-Dimethylcarbamoyl-2,2,6,6-tetramethylpiperidin | 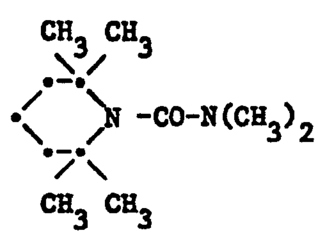 | Smp. 69 - 70° |

| | | | |
|---|---|---|---|
| 3 | 1-Diethylcarbamoyl-2,2,6,6-tetramethylpiperidin | 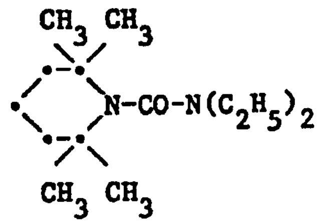 | Smp. 32 - 34° |
| 4 | 1-Diethylcarbamoyl-2,2,6,6-tetramethyl-4-benzoyloxypiperidin | 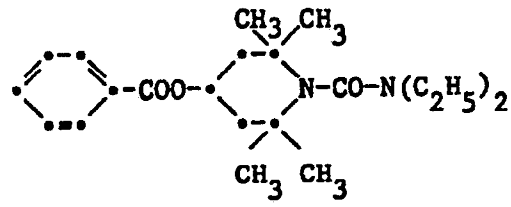 | Smp. 44 - 46° |
| 5 | 1-Diethylcarbamoyl-2,2,6,6-tetramethylpiperidon-4 | 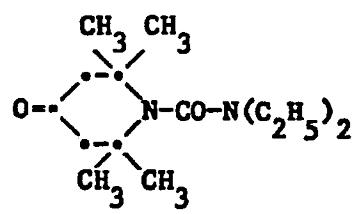 | Smp. 80 - 82° |
| 6 | 1-Di-(2-hydroxyethyl)-carbamoyl-2,2,6,6-tetra-methylpiperidin | 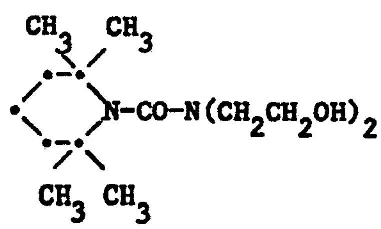 | Öl |
| 7 | 3-Ethyl-3-acetoxymethyl-8,8,10,10-tetramethyl-9-diethylcarbamoyl-1,5-dioxa-9-aza-spiro[5.5]decan | 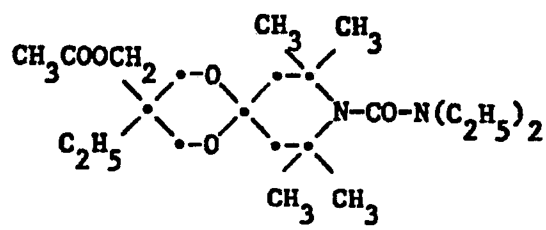 | Smp. 80 - 81° |
| 8 | N,N'-Dimethyl-N,N'-bis-(2,2,6,6-tetramethylpiperidincarbonyl)-ethylendiamin | 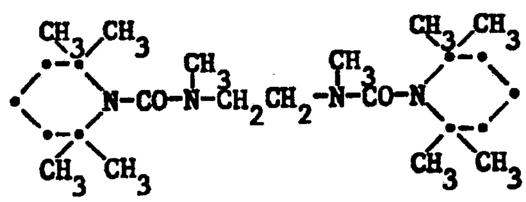 | Smp. 234 - 236° |
| 9 | 8-Morpholinocarbonyl-1,3,8-triaza-2,4-dioxo-3-dodecyl-7,7,9,9-tetramethylspiro[4,5]-decan | 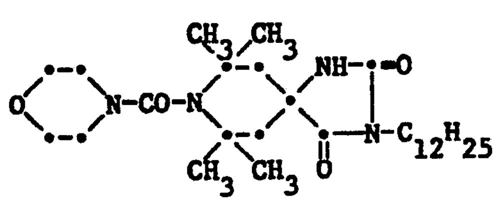 | Smp. 152 - 153° |

| | | | |
|---|---|---|---|
| 10 | 1-Piperidinocarbonyl-4-(β-piperidinopropionoxy)-2,2,6,6-tetramethylpiperidin | 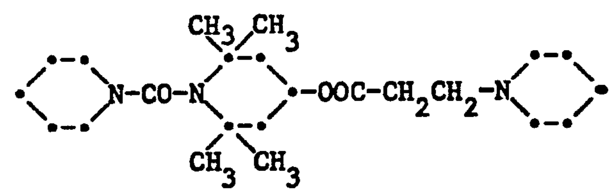 | Smp. 84 - 86° |

**Beispiel 2**

**Carbamoylierung in Toluol.**

Zu einer Phosgenlösung in Toluol (24,7 ml einer 20 %-igen Lösung entsprechend 0,05 Mol Phosgen) tropft man bei -30° innerhalb einer Stunde die Lösung von 14,2 g (0,1 Mol) 2,2,6,6-Tetramethylpiperidin in 25 ml Toluol. Nach weiteren 2 Std. Rühren bei 0° werden nun innerhalb 2 Std. 26,7 g (0,11 Mol) Di-n-octylamin in 25 ml Toluol zugetropft und anschliessend 16 Std. bei Raumtemperatur gerührt. Zur Aufarbeitung wird vom ausgefallenen Salz abfiltriert, das Filtrat wiederholt mit wenig Wasser gewaschen, über Natriumsulfat getrocknet und das Lösungsmittel im Wasserstrahlvakuum vollständig abdestilliert. Die rohe Verbindung wird durch Säulenchromatographie an Kieselgel weiter gereinigt (Eluierungsmittel: Hexan-Diethylether 9 : 1), wodurch das reine 1-Di-n-octylcarbamoyl-2,2,6,6-tetramethylpiperidin (Verbindung Nr. 11) als viskose Flüssigkeit erhalten wird. $n^{20}_D$: 1,4728.

Das $^1$H-NMR-Spektrum der erhaltenen Verbindung steht mit der angegebenen Struktur in Einklang.

Elementaranalyse:

| | | | |
|---|---|---|---|
| $C_{26}H_{52}N_2O$ | Ber.: C 76,41 | H 12,83 | N 6,85 % |
| (408,7) | Gef.: C 76,5 | H 12,6 | N 6,8 % |

**Beispiel 3**

**Carbamoylierung unter Zusatz einer Hilfsbase**

Zu einer Lösung von 84,5 g (0,4 Mol) 4-Acryloyloxy-2,2,6,6-tetramethylpiperidin, 0,2 g Di-tert.butyl-p-kresol und 129,3 g (1,0 Mol) Diisopropylethylamin in 300 ml Essigsäureethylester wird unter Rühren bei 0 - 5°C innerhalb von ca. 6 Std. die Lösung von 39,6 g (0,4 Mol) Phosgen in ca. 300 ml Essigsäureethylester getropft. Nach weiteren 3 Std. Rühren bei Raumtemperatur tropft man bei ca. 20° innert einer Std. (unter leichter Aussenkühlung und kräftigem Rühren) die Lösung von 35,7 g (0,41 Mol) Morpholin in 40 ml Essigsäureethylester zu der weissen Suspension. Nach weiteren 6 Std. Rühren bei Raumtemperatur wird das Reaktionsgemisch filtriert, der Salzrückstand mit Hexan gut ausgewaschen und das Filtrat im Vakuumrotationsverdampfer von den Lösungsmitteln und dem überschüssigen Diisopropylethylamin befreit. Den Rückstand löst man in Methylenchlorid, wascht diese Lösung zweimal mit Wasser, dreimal mit kalter n-Salzsäure und wieder zweimal mit Wasser, trocknet die organische Phase über Natriumsulfat und destilliert das Lösungsmittel im Vakuum ab. Das kristallin erstarrende Rohprodukt wird in Diisopropylether umkristallisiert, wodurch das reine 1-Morpholinocarbonyl-4-acryloyloxy-2,2,6,6-tetramethylpiperidin vom Smp. 129 - 130° erhalten wird (Verbindung Nr. 12).

Elementaranalyse:

| | | | |
|---|---|---|---|
| $C_{17}$- $H_{28}N_2O_4$ | Ber.: C 62,94 | H 8,70 | N 8,64 % |
| (324,4) | Gef.: C 63,2 | H 8,8 | N 8,4 % |

Das $^1$H-NMR steht mit der angegebenen Struktur in Einklang.

Analog wird hergestellt 1-Piperidinocarbonyl-4-acryloyloxy-2,2,6,6-tetramethylpiperidin vom Smp. 90 - 92° (Verbindung Nr. 13).

**Beispiel 4**

**Hydrolyse zum 4-Hydroxypiperidin**

Eine Lösung von 41,8 g 1-Diethylcarbamoyl-4-acetoxy-2,2,6,6-tetramethylpiperidin (0,14 Mol) (Verbindung Nr. 1) in 200 ml Methanol wird mit einer Lösung von 5,7 g Natriumhydroxid in 50 ml Methanol versetzt. Nach 16 Std. Rühren bei Raumtemperatur wird das Reaktionsgemisch im Vakuumrotationsverdampfer vom Methanol befreit, der Rückstand in Methylenchlorid gelöst und dreimal mit Wasser gewaschen. Die organische Phase wird über Natriumsulfat getrocknet, filtriert und das Methylenchlorid abdestilliert. Der kristalline Rückstand wird in Diisopropylether umkristallisiert, wodurch

reines 1-Diethylcarbamoyl-4-hydroxy-2,2,6,6-tetramethylpiperidin (Verbindung Nr. 14) vom Smp. 118 - 119° erhalten wird.

| Elementaranalyse: | | | |
|---|---|---|---|
| $C_{14}H_{28}N_2O_2$ | Ber.: C 65,59 | H 11,01 | N 10,93 % |
| (256,4) | Gef.: C 65,6 | H 10,8 | N 11,0 % |

Das $^1$H-NMR-Spektrum steht mit der angegebenen Struktur der erhaltenen Verbindung in Einklang.

Analog wird durch Hydrolyse der Verbindung Nr. 9 das 1-Morpholinocarbonyl-4-hydroxy-2,2,6,6-tetramethylpiperidin vom Smp. 142 - 143° (Verbindung Nr. 15) und durch Hydrolyse der Verbindung Nr. 10 das 1-Piperidinocarbonyl-4-hydroxy-2,2,6,6-tetramethylpiperidin (Verbindung Nr. 16) hergestellt.

**Beispiel 5**

**Umsetzung von 4-Hydroxypiperidinen**

Eine Lösung von 15,4 g 1-Diethylcarbamoyl-4-hydroxy-2,2,6,6-tetramethylpiperidin (0,06 Mol) (Verbindung Nr. 14) und 6,9 g (0,03 Mol) Sebacinsäuredimethylester in 200 ml Xylol werden mit 0,1 ml Tetrabutylorthotitanat versetzt und im leichten Stickstoffstrom langsam bis zu einer Maximaltemperatur von 145° erhitzt, wobei das gebildete Methanol fortlaufend und schliesslich auch das Xylol langsam innerhalb von 8 St. vollständig ausdestilliert wird. Nach dem Abkühlen wird das Reaktionsgemisch in Methylenchlorid gelöst, mit Wasser gewaschen, über Natriumsulfat getrocknet und das Lösungsmittel abdestilliert. Die kristallin erstarrende, rohe Verbindung wird in Pentan umkristallisiert, wodurch reines Bis-(1-diethylcarbamoyl-2,2,6,6-tetramethylpiperidinyl-4)-sebacat (Verbindung Nr. 17) vom Smp. 72 - 73° erhalten wird.

| Elementaranalyse: | | | |
|---|---|---|---|
| $C_{38}H_{70}N_4O_6$ | Ber.: C 67,22 | H10,39 | N 8,25 % |
| (679,0) | Gef.: C 67,0 | H 10,5 | N 8,1 % |

Das $^1$H-NMR-Spektrum der erhaltenen Verbindung ist mit der angegebenen Struktur gut verträglich.

Analog wird durch Umsetzung der Verbindung Nr. 14 mit Dimethyladipat das Bis-(1-diethylcarbamoyl-2,2,6,6-tetramethylpiperidinyl-4)-adipat vom Smp. 104 - 105° hergestellt (Verbindung Nr. 18).

Durch analoge Umsetzung der Verbindung Nr. 16 mit einem Überschuss an Diethylcarbonat und anschliessend mit Hexandiol-1,6 im Mol-Verhältnis 2 : 1 erhält man das Hexamethylen-bis(1-piperidinocarbonyl-2,2,6,6-tetramethylpiperidinyl-4)-carbonat (Verbindung Nr. 19) das bei 128 - 130° schmilzt.

Durch Umsetzung der Verbindung Nr. 15 mit Hexamethylendiisocyanat erhält man das O,O'-Bis(1-morpholinocarbonyl-2,2,6,6-tetramethyl-4-piperidinyl)-N,N'-hexamethylendicarbamat (Verbindung Nr.20), das bei 128°-130° und bei 202 - 204° schmilzt.

**Beispiel 6**

**Polymerisation**

Zu einer auf 78°C erhitzten Lösung von 11,4 g (0,035 Mol) 1-Morpholinocarbonyl-4-acryloyloxy-2,2,6,6-tetramethylpiperidin (Verbindung Nr. 12) in 45 ml Benzol lässt man unter Rühren innerhalb von 5 Min. die Lösung von 40 mg Azobisisobutyronitril in 5 ml Benzol zufliessen. Anschliessend wird die radikalische Polymerisation während 7 Std. bei 78° weitergeführt. Nach dem Abdestillieren von ca. 35 ml Benzol im Vakuum wird das Polymerkonzentrat bei Raumtemperatur unter kräftigem Turbinieren langsam in 200 ml Diethylether eingegossen, wodurch das Polymere als weisses Pulver ausgefällt wird. Die Fällung wird abfiltriert, mit Diethylether sorgfältig nachgewaschen und im Vakuum bei 60° getrocknet. Das so erhaltene farblose, pulverige Poly-1-morpholinocarbonyl-4-acryloyloxy-2,2,6,6-tetramethylpiperidin besitzt einen Erweichungspunkt ($T_S$) von 185 - 190° und ein mittleres Molekulargewicht ($\bar{M}_n$) von 3400. (Verbindung Nr. 21).

Analog wird durch Polymerisation der Verbindung Nr. 13 das Poly-1-piperidinocarbonyl-4-acryloyloxy-2,2,6,6-tetramethylpiperidin (Verbindung Nr. 22) hergestellt, das bei 160° erweicht und ein $\bar{M}_n$ von 3200 besitzt.

**Beispiel 7**

**Stabilisierung von Polypropylenfolien**

100 Teile Polypropylenpulver (Moplen®, Fibre grade, der Firma Montedison) werden mit 0,2 Teilen β-(3,5-Di-tert.butyl-4-hydroxyphenyl)-propionsäure-octadecylester und 0,25 Teilen eines Lichtschutzmittels der folgenden Tabelle 2 im Brabender-Plastographen bei 200°C während 10 Minuten homogenisiert. Die so erhaltene Masse wird möglichst rasch dem Kneter entnommen und in einer Kniehebelpresse zu einer 2 - 3 mm dicken Platte gepresst. Ein Teil des erhaltenen Presslings wird ausgeschnitten und zwischen zwei Hochglanz-Hartaluminiumfolien mit einer hydraulischen Laborpresse während 6 Minuten bei 260° und 12 Tonnen Druck zu einer 0,1 mm dicken Folie gepresst, die 1 Stunde bei 150°C getempert und anschliessend unverzüglich in kaltem Wasser abgeschreckt wird. Aus dieser werden nun Abschnitte ausgestanzt und im Xenotest® 1200 belichtet. Zu regelmässigen Zeitabständen werden diese Prüflinge aus dem Belichtungsapparat entonmmen und in einem IR-Sprektrophotometer auf ihren Carbonylgehalt geprüft. Die Zunahme der Carbonylextinktion bei 5,85 μ während der Belichtung ist ein Mass für den photoxidativen Abbau des Polymeren (s. L. Balaban et al., J. Polymer Sci, Part C; *22*, 1059 - 1071 (1969) und ist erfahrungsgemäss mit einem Abfall der mechanischen Eigenschaften des Polymeren verbunden. Als Mass der Schutzwirkung gilt die Zeit bis Erreichen einer Carbonylextinktion von ca. 0,3, bei welcher die Folie brüchig ist.

**Tabelle 2**

| Lichtschutzmittel | Belichtungszeit bis Carbonylextinktion 0,3 |
|---|---|
| keines | 890 h |
| Verbindung Nr. 8 | 2385 h |
| Verbindung Nr. 9 | >3360 h |
| Verbindung Nr. 10 | >3200 h |
| Verbindung Nr. 17 | 6080 h |
| Verbindung Nr. 18 | >5540 h |
| Verbindung Nr. 20 | >3400 h |
| Verbindung Nr. 21 | 2170 h |
| Verbindung Nr. 22 | 2080 h |

**Beispiel 8**

**Stabilisierung eines 2-Schicht-Metalleffekt-Lackes**

Aluminiumbleche von 0,5 mm Stärke werden mit einem Aluminium pigmentierten Grundlack auf Basis Polyester/Celluloseacetobutyrat/Melaminharz beschichtet. Auf den nassen Grundlack wird ein Klarlack der folgenden Zusammensetzung aufgespritzt:

58,3 Teile Viacryl® VC 373 (Acrylharz der Fa. Vianova, Wien)
27,3 Teile Maprenal® MF 590 (Melaminharz der Fa. Höchst AG, Frankfurt)
1,0 Teil 1 %-ige Lösung eines Silikonharzes in Xylol
4,0 Teile Solvesso® 150 (aromatisches Lösungsmittelgemisch)
5,4 Teile Xylol
4,0 Teile Ethylglykolacetat.

Dazu kommen 0,9 Teile des in der Tabelle 3 angegebenen Lichtschutzmittels. Dieser Klarlack hat eine Viskosität von 21 sec/DIN-Becher 4.

Er wird in einer Schichtdicke von 40 μm aufgetragen und bei 130°C 39 Minuten eingebrannt.

Die Proben werden in einem UVCON - Schnellbewitterungsgerät der Fa. Atlas mit einem Zyklus von 4 h UV-Bestrahlung bei 60° und 4 h Bewitterung bei 50° 2000 h bewittert. Nach 1000 h und nach 2000 h wird der 20°-Glanz gemäss DIN 67530 gemessen. Ausserdem werden die Proben in regelmässigen Abständen unter dem Stereomikroskop auf Rissbildung untersucht. Die Ergebnisse sind in Tabelle 3 zusammengestellt.

**Tabelle 3**

| Lichtschutzmittel | 20°-Glanz nach | | | Rissbildung |
|---|---|---|---|---|
| | 0 | 1000 | 2000 h | bemerkbar |
| keines | 97 | 47 | 9 | nach 1600 h |
| Verbindung Nr. 7 | 92 | 75 | 54 | keine |
| Verbindung Nr. 8 | 96 | 64 | 36 | keine |
| Verbindung Nr. 17 | 94 | 78 | 63 | keine |
| Verbindung Nr. 18 | 89 | 59 | 57 | keine |

**Patentansprüche**

1. Verbindungen der Formel I,

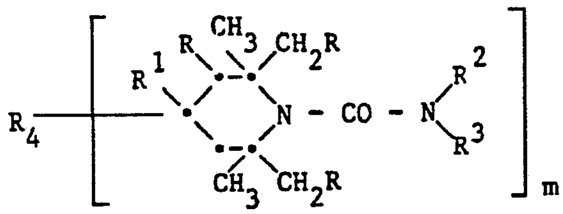

I

worin
m eine ganze Zahl von 1 bis 4 ist,
R Wasserstoff oder $C_1$-$C_4$-Alkyl ist,
$R^1$ Wasserstoff, $C_2$-$C_{12}$-Alkoxy, $C_2$-$C_{20}$-Alkanoyloxy, Benzyloxy, $C_3$-$C_{25}$-Carbamoyloxy oder CN ist,
$R^2$ $C_1$-$C_{18}$-Alkyl, $C_3$-$C_{12}$-Alkoxyalkyl, $C_2$-$C_8$-Hydroxyalkyl, $C_3$-$C_{12}$-Alkenyl, $C_7$-$C_{14}$-Aralkyl, $C_6$-$C_{14}$-Aryl, $C_7$-$C_{14}$-Alkaryl, $C_3$-$C_7$-Cycloalkyl oder 2,2,6,6-Tetramethylpiperidin-4-yl ist,
$R^3$ eine der für $R^2$ gegebenen Bedeutungen hat oder
$R^2$ und $R^3$ zusammen mit dem N-Atom, an das sie gebunden sind, einen 5 - 7-gliedrigen heterocyclischen Ring bilden und
$R^4$ Wasserstoff oder einen m-wertigen organischen Rest bedeutet oder
$R^1$ und R zusammen einen Oxo-Sauerstoff oder einen zweiwertigen organischen Rest bedeuten.

2. Verbindungen gemäss Anspruch 1 der Formel III,

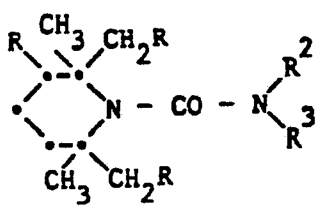

III

worin R, $R^2$ und $R^3$ die in Anspruch 1 gegebene Bedeutung haben.

3. Verbindungen gemäss Anspruch 1 der Formel IV,

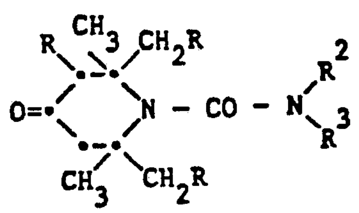

IV

worin R, $R^2$ und $R^3$ die in Anspruch 1 gegebene Bedeutung haben.

4. Verbindungen gemäss Anspruch 1 der Formel V

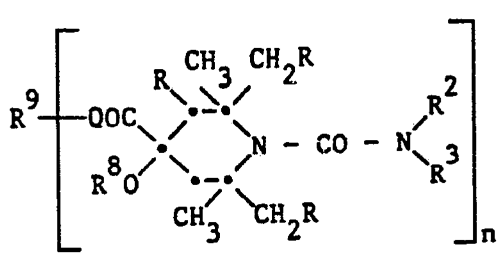

V

worin n 1 - 4 ist, $R^8$ $C_1$-C-Alkyl oder $C_2$-$C_{12}$-Alkanoyl ist, $R^9$ der n-wertige Rest eines $C_1$-$C_{20}$-Alkohols, $C_2$-$C_{16}$-Diols,

$C_3$-$C_{18}$-Triols oder $C_4$-$C_{20}$-Tetrols ist, der durch ein oder mehrere Sauerstoffatome unterbrochen sein kann, und R, $R^2$ und $R^3$ die in Anspruch 1 gegebene Bedeutung hat.

5. Verbindungen gemäss Anspruch 1 der Formel VI,

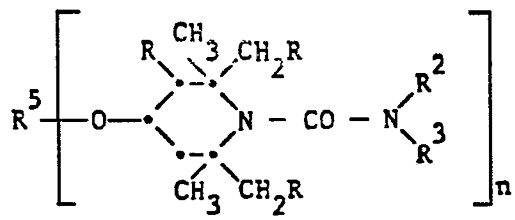

VI

worin n eine ganze Zahl von 1 - 4 ist,
$R^5$, wenn n = 1 ist, Wasserstoff, wenn n = 2 ist, $C_2$-$C_{12}$-Alkylen, $C_4$-$C_{12}$-Alkenylen, Xylylen, einen zweiwertigen Acylrest einer aliphatischen oder cycloaliphatischen Dicarbonsäure, Dicarbaminsäure oder Phosphor enthaltenden Säure, wenn n = 3 ist, einen dreiwertigen Acylrest einer aliphatischen, cycloaliphatischen, araliphatischen, aromatischen oder heterocyclischen Tricarbonsäure, Tricarbaminsäure oder Phosphor enthaltenden Säure, wenn n = 4 ist, einen vierwertigen Acylrest einer aliphatischen, cycloaliphatischen oder aromatischen Tetracarbonsäure bedeutet und R, $R^2$ und $R^3$ die in Anspruch 1 gegebene Bedeutung haben.

6. Verbindungen gemäss Anspruch 1 der Formel VII,

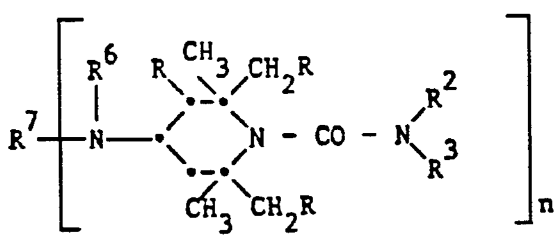

VII

worin n 1 oder 2 ist,
$R^6$ $C_1$-$C_{12}$-Alkyl, $C_3$-$C_{12}$-Alkoxyalkyl, $C_5$-$C_7$-Cycloalkyl, $C_7$-$C_9$-Aralkyl, $C_2$-$C_{18}$-Alkanoyl, $C_3$-$C_5$-Alkenoyl, Benzoyl, $C_2$-$C_{13}$-Alkoxycarbonyl oder $C_7$-$C_{11}$-Aryloxycarbonyl bedeutet, und
$R^7$, wenn n = 1 ist, H, $C_1$-$C_{12}$-Alkyl, $C_3$-$C_{12}$-Alkoxyalkyl, $C_5$-$C_8$-Cycloalkyl, $C_3$-$C_8$-Alkenyl, Glycidyl oder Cyanoethyl, und wenn n = 2 ist, $C_2$-$C_{12}$-Alkylen, $C_6$-$C_{15}$-Arylen, Xylylen, eine Gruppe $-CH_2-CH(OH)-CH_2$ oder $-CH_2 \cdot CH(OH)-CH_2-O-D-O-CH_2-CH(OH)-CH_2-$ bedeutet, worin D $C_2$-$C_{10}$-Alkylen, $C_6$-$C_{15}$-Arylen oder $C_6$-$C_{12}$-Cycloalkylen ist, oder im Falle dass $R_6$ Alkyl, Cycloalkyl oder Aralkyl ist,
$R^7$ auch ein zweiwertiger Acylrest einer aliphatischen, cycloaliphatischen oder aromatischen Dicarbonsäure oder Dicarbaminsäure sein kann, oder $R^6$ und $R^7$ zusammen mit dem N-Atom im Falle von n = 1 ein Imidrest einer aliphatischen, cycloaliphatischen oder aromatischen 1,2-Dicaronsäure mit 4 - 12 C-Atomen sind.

7. Verbindungen gemäss Anspruch 1 der Formel VIII,

VIII

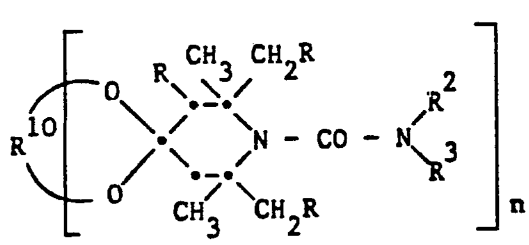

worin n 1 oder 2 ist, $R^{10}$ im Falle von n = 1 $C_2$-$C_8$-Alkylen oder -Hydoxyalkylen oder $C_4$-$C_{22}$-Acyloxyalkylen und im Falle von n = 2 die Gruppe $(-CH_2)_2C(CH_2-)_2$ bedeutet und R, $R^2$ und $R^3$, die im Anspruch 1 gegebene Bedeutung haben.

8. Verbindunge gemäss Anspruch 1 der Formel IX,

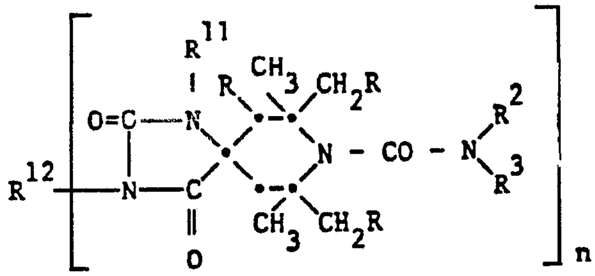

IX

worin n 1 oder 2 ist, R Wasserstoff, $C_1$-$C_{12}$-Alkyl, Allyl, Benzyl, Glycidyl oder $C_2$-$C_6$-Alkoxyalkyl ist, $R^{12}$ bei n = 1 Wasserstoff, $C_1$-$C_{12}$-Alkyl, $C_3$-$C_5$-Alkenyl, $C_{7-9}$-Aralkyl, $C_5$-$C_8$-Cycloalkyl, $C_2$-$C_4$-Hydroxyalkyl, $C_3$-$C_6$-Alkoxyalkyl, $C_6$-$C_{10}$-Aryl oder Glycidyl und bei n = 2 $C_2$-$C_{12}$-Alkylen, $C_6$-$C_{15}$-Arylen, $C_4$-$C_8$-Alkenylen oder eine Gruppe $-CH-CH(OH)-CH_2-O-D-O-CH_2-CH(OH)-CH_2-$ bedeuten, worin D $C_2$-$C_{12}$-Alkylen, $C_6$-$C_{15}$-Arylen oder $C_6$-$C_{12}$-Cycloalkylen ist, und R, $R^2$ und $R^3$ die in Anspruch 1 gegebene Bedeutung haben.

9. Verbindungen gemäss Anspruch 1 der Formel XII

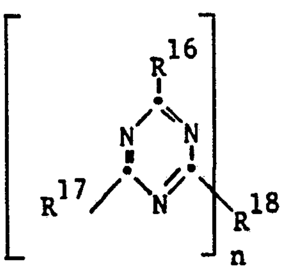

XII

worin n 1 oder 2 ist, $R^{16}$ eine Gruppe der Formel

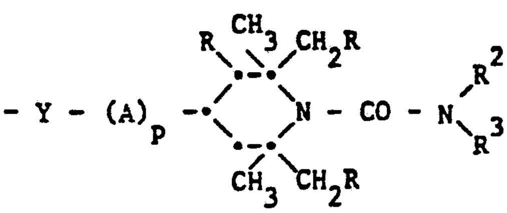

ist, worin R, $R^2$ und $R^3$ die in Anspruch 1 gegebene Bedeutung haben, Y -O- oder $-NR^{19}-$ ist, $R^{19}$ H, $C_1$-$C_{12}$-Alkyl, $C_2$-$C_4$-Hydroxyalkyl, $C_3$-$C_6$-Alkoxyalkyl, Cyclohexyl, Benzyl oder eine Gruppe

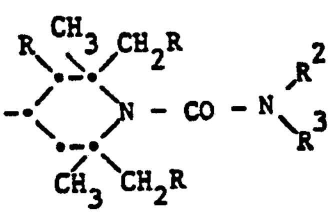

darstellt, A $C_2$-$C_6$-Alkylen oder $-(CH_2)_3-O-$ und p null oder 1 bedeuten,
$R^{17}$ eine der für $R^{16}$ gegebenen Bedeutungen hat oder $-NR^{20}R^{21}$, $-OR^{22}$, $-NHCH_2OR-$ oder $-N(CH_2OR^{23})_2$

bedeutet, worin $R^{20}$ eine der für $R^{19}$ gegebenen Bedeutungen hat, $R^{21}$ $C_1$-$C_{12}$-Alkyl, Cyclohexyl oder Benzyl ist, oder $R^{20}$ und $R^{21}$ zusammen $C_4$-$C_5$-Alkylen oder Oxaalkylen sind,

$R^{22}$ Wasserstoff, -$C_1$-$C_{12}$-Alkyl oder Phenyl ist und $R^{23}$ Wasserstoff oder $C_1$-$C_4$-Alkyl bedeutet, und

$R^{18}$ bei n = 1 eine der für $R^{16}$ und $R^{17}$ gegebenen Bedeutungen hat und bei n = 2 eine Gruppe -Y-Q-Y- darstellt, worin Q $C_2$-$C_{12}$-Alkylen, durch -O-, -NH-, -N-Alkyl oder durch eine Gruppe der Formel

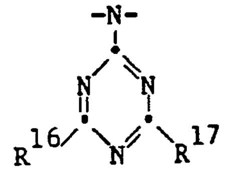

unterbrochenes $C_4$-$C_{12}$-Alkylen, Cyclohexylen, Xylylen oder Phenylen bedeutet.

10. Verbindungen gemäss Anspruch 1 der Formel XIII,

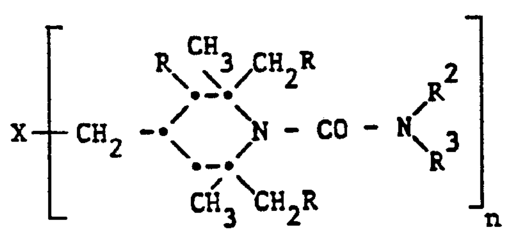

XIII

worin n 1 oder 2 ist,

X, wenn n = 1 ist, -CN, -$COOR^{24}$ , -$CH_2NH_2$, -$CH_2OH$ oder -$CH_2COOR^{24}$ bedeutet, worin $R^{24}$ $C_1$-$C_{18}$-Alkyl, Benzyl oder Cycloalkyl bedeutet, und wenn n = 2 ist, -CO-O-$R^{25}$ -O-CO- bedeutet, worin $R^{25}$ $C_2$-$C_{12}$-Alkylen, durch -O- oder-N($C_1$-$C_4$-Alkyl)- unterbrochenes $C_4$-$C_{10}$-Alkylen, $C_6$-$C_{15}$-Cycloalkylen, p-Xylylen oder Hexahydroxylylen bedeutet und R, $R^2$ und $R^3$ die in Anspruch 1 gegebenen Bedeutungen haben.

11. Verfahren zur Herstellung von Verbindungen der Formel I gemäss Anspruch 1 durch Umsetzung von Verbindungen der Formel II

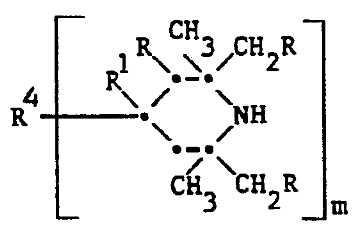

II

mit Phosgen in einem inerten Lösungsmittel in Gegenwart von molaren Mengen einer Base und anschliessende Umsetzung mit einem sekundären Amin der Formel $R^2$-NH-$R^3$ ebenfalls in Gegenwart von molaren Mengen einer Base, wobei als Base in der ersten Stufe auch ein Überschuss der Verbindung II und in der zweiten Stufe auch ein Überschuss der Verbindung $R^2$-NH-$R^3$ verwendet werden kann.

12. Verfahren gemäss Anspruch 11, wonach man pro Mol II 0,9 - 1,2 Mol Phosgen, mindestens ein Mol des sekundären Amines und mindestens 2 Mol einer Base verwendet.

13. Verfahren gemäss Anspruch 11, dadurch gekennzeichnet, dass man als Base ein tertiäres Amin verwendet.

14. Verfahren gemäss Anspruch 11, dadurch gekennzeichnet, dass man die gesamte Umsetzung bei Temperaturen unterhalb 40°C ausführt.

15. Verfahren gemäss Anspruch 11 zur Herstellung von Verbindungen der Formel I, worin R Wasserstoff ist.

16. Verfahren gemäss Anspruch 11 zur Herstellung von Verbindungen der Formel I, worin R' Wasserstoff ist.

17. Verwendung der Verbindungen des Anspruches 1 als Stabilisatoren für organische Materialien, insbesondere für organische Polymere.

18. Organisches Polymer, enthaltend als Stabilisator 0,01 bis 5 Gew.-% einer Verbindung des Anspruches 1.

**Claims**

1. A compound of the formula I

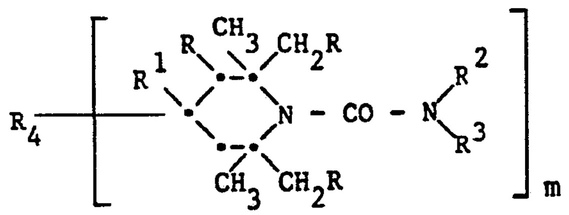

I

in which m is an integer from 1 to 4,
R is hydrogen or $C_1$-$C_4$alkyl,
$R^1$ is hydrogen, $C_2$-$C_{12}$-alkoxy, $C_2$-$C_{20}$alkanoyloxy, benzyloxy, $C_2$-$C_{25}$carbamoyloxy or CN,
$R^2$ is $C_1$-$C_{18}$alkyl, $C_3$-$C_{12}$alkoxyalkyl, $C_2$-$C_8$hydroxyalkyl, $C_3$-$C_{12}$alkenyl, $C_7$-$C_{14}$aralkyl, $C_6$-$C_{14}$aryl, $C_7$-$C_{14}$alkaryl, $C_3$-$C_7$cycloalkyl or 2,2,6,6-tetramethylpiperidin-4-yl,
$R^3$ has one of the meanings of $R^2$, or $R^2$ and $R^3$, together with the N atom to which they are bonded, form a 5- to 7-membered heterocyclic ring, and
$R^4$ is hydrogen or an m-valent organic radical, or
$R^1$ and $R^4$ together are an oxo-oxygen or a divalent organic radical.

2. A compound according to claim 1 of the formula III

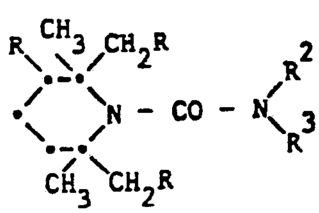

III

in which R, $R^2$ and $R^3$ are as defined in claim 1.

3. A compound according to claim 1 of the formula IV

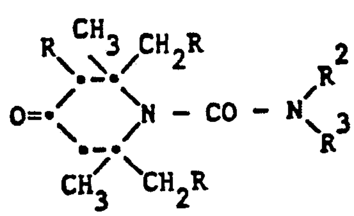

IV

in which R, $R^2$ and $R^3$ are as defined in claim 1.

4. A compound according to claim 1 of the formula V

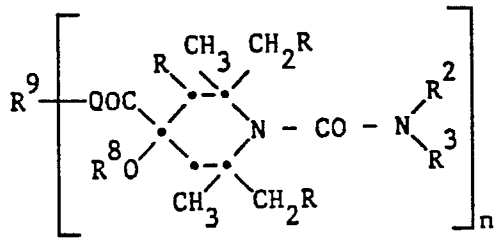

V

in which n is 1 to 4, $R^8$ is $C_1$-$C_4$alkyl or $C_2$-$C_{12}$alkanoyl, $R^9$ is the n-valent radical of a $C_1$-$C_{20}$alcohol, $C_2$-$C_{16}$diol, $C_3$-$C_{18}$triol or $C_4$-$C_{20}$tetrol, which can be interrupted by one or more oxygen atoms, and R, $R^2$ and $R^3$ are as defined in claim 1.

5. A compound according to claim 1 of the formula VI

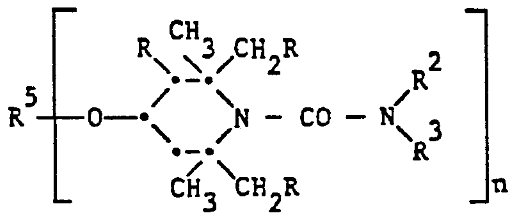

VI

in which n is an integer from 1 to 4, and, if n is 1, $R^5$ is hydrogen, or, if n is 2, $R^5$ is $C_2$-$C_{12}$alkylene, $C_4$-$C_{12}$alkenylene, xylylene, or a divalent acyl radical of an aliphatic or cycloaliphatic dicarboxylic acid, dicarbamic acid or phosphorus-containing acid, or, if n is 3, $R^5$ is a trivalent acyl radical of an aliphatic, cycloaliphatic, araliphatic, aromatic or heterocyclic tricarboxylic acid, tricarbamic acid or phosphorus-containing acid, or, if n is 4, $R^5$ is a tetravalent acyl radical of an aliphatic, cycloaliphatic or aromatic tetracarboxylic acid, and R, $R^2$ and $R^3$ are as defined in claim 1.

6. A compound according to claim 1 of the formula VII

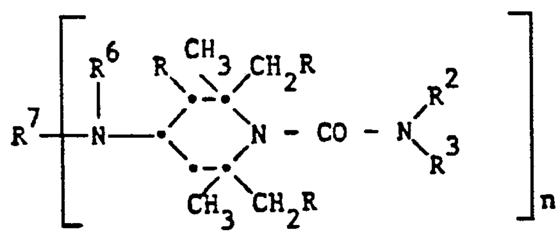

VII

in which n is 1 or 2,
$R^6$ is $C_1$-$C_{12}$alkyl, $C_3$-$C_{12}$alkoxyalkyl, $C_5$-$C_7$cycloalkyl, $C_7$-$C_9$aralkyl, $C_2$-$C_{18}$alkanoyl, $C_3$-$C_5$alkenoyl, benzoyl, $C_2$-$C_{13}$alkoxycarbonyl or $C_7$-$C_{11}$aryloxycarbonyl and, if n is 1,
$R^7$ is H, $C_1$-$C_{12}$alkyl, $C_3$-$C_{12}$alkoxyalkyl, $C_5$-$C_8$cycloalkyl, $C_3$-$C_8$alkenyl, glycidyl or cyanoethyl, or, if n is 2, $R^7$ is $C_2$-$C_{12}$alkylene, $C_6$-$C_{15}$arylene, xylylene, or a $-CH_2-CH(OH)-CH_2-$ or $-CH_2CH(OH)-CH_2-O-D-O-CH_2-CH(OH)-CH_2-$ group, in which D is $C_2$-$C_{10}$alkylene, $C_6$-$C_{15}$arylene or $C_6$-$C_{12}$cycloalkylene, or, in the case where $R^6$ is alkyl, cycloalkyl or aralkyl, $R^7$ may also be a divalent acyl radical of an aliphatic, cycloaliphatic or aromatic dicarboxylic acid or dicarbamic acid, or, if n is 1,
$R^6$ and $R^7$, together with the N atom, are an imide radical of an aliphatic, cycloaliphatic or aromatic 1,2-dicarboxylic acid having 4 - 12 C atoms.

7. A compound according to claim 1 of the formula VIII

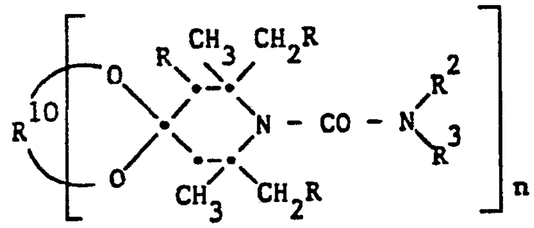

VIII

in which n is 1 or 2, and, if n is 1, $R^{10}$ is $C_2$-$C_8$alkylene, $C_2$-$C_8$hydroxyalkylene or $C_4$-$C_{22}$acyloxyalkylene, or, if n is 2, $R^{10}$ is the $(-CH_2)_2C(CH_2-)_2$ group, and R, $R^2$ and $R^3$ are as defined in claim 1.

8. A compound according to claim 1 of the formula IX

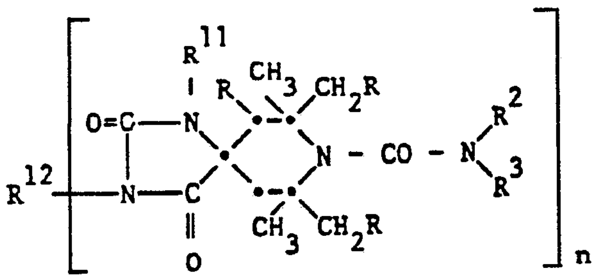

IX

in which n is 1 or 2,
$R^{11}$ is hydrogen, $C_1$-$C_{12}$alkyl, allyl, benzyl, glycidyl or $C_2$-$C_6$alkoxyalkyl, and, if n is 1, $R^{12}$ is hydrogen, $C_1$-$C_{12}$alkyl, $C_3$-$C_5$alkenyl, $C_7$-$C_9$aralkyl, $C_5$-$C_8$cycloalkyl, $C_2$-$C_4$hydroxyalkyl, $C_3$-$C_6$alkoxyalkyl, $C_6$-$C_{10}$aryl or glycidyl, or, if n is 2, $R^{12}$ is $C_2$-$C_{12}$alkylene, $C_6$-$C_{15}$arylene, $C_4$-$C_8$alkenylene or a -CH-CH(OH)-$CH_2$-O-D-O-$CH_2$-CH(OH)-$CH_2$- group, in which D is $C_2$-$C_{12}$alkylene, $C_6$-$C_{15}$arylene or $C_6$-$C_{12}$cycloalkylene, and R, $R^2$ and $R^3$ are as defined in claim 1.

9. A compound according to claim 1 of the formula XII

XII

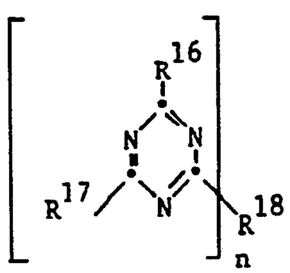

in which n is 1 or 2 and $R^{16}$ is a group of the formula

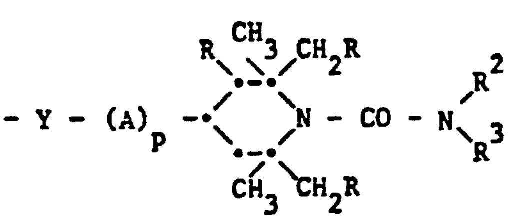

in which R, $R^2$ and $R^3$ are as defined in claim 1, Y is -O- or -$NR^{19}$-, $R^{19}$ is H, $C_1$-$C_{12}$alkyl, $C_2$-$C_4$hydroxyalkyl, $C_3$-$C_6$alkoxyalkyl, cyclohexyl, benzyl or a group

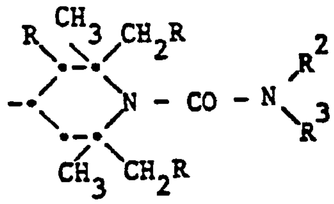

A is $C_2$-$C_6$alkylene or $-(CH_2)_3$-O- and p is zero or 1, $R^{17}$ has one of the meanings given for $R^{16}$ or is $-NR^{20}R^{21}$, $-OR^{22}$, $-NHCH_2OR^{23}$ or $-N(CH_2OR^{23})_2$, in which $R^{20}$ has one of the meanings given for $R^{19}$ and $R^{21}$ is $C_1$-$C_{12}$alkyl, cyclohexyl or benzyl, or $R^{20}$ and $R^{21}$ together are $C_4$-$C_5$alkylene or oxaalkylene, $R^{22}$ is hydrogen, $C_1$-$C_{12}$alkyl or phenyl and $R^{23}$ is hydrogen or $C_1$-$C_4$alkyl, and, if n is 1, $R^{18}$ has one of the meanings given for $R^{16}$ and $R^{17}$, or, if n is 2, $R^{18}$ is a -Y-Q-Y- group, in which Q is $C_2$-$C_{12}$alkylene, $C_4$-$C_{12}$alkylene which is interrupted by -O-, -NH-, -N-alkyl or by a group of the formula

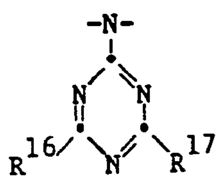

or Q is cyclohexylene, xylylene or phenylene.

10. A compound according to claim 1 of the formula XIII

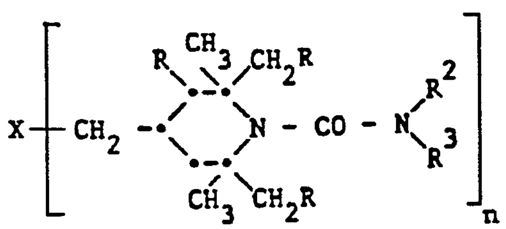

XIII

in which n is 1 or 2, and, if n is 1, X is -CN, $-COOR^{24}$, $-CH_2NH_2$, $-CH_2OH$ or $-CH_2COOR^{24}$, in which $R^{24}$ is $C_1$-$C_{18}$alkyl, benzyl or cycloalkyl, or, if n is 2, X is $-CO-O-R^{25}-O-CO-$, in which $R^{25}$ is $C_2$-$C_{12}$alkylene, $C_4$-$C_{10}$alkylene which is interrupted by -O- or $-N(C_1$-$C_4$alkyl)-, or $C_6$-$C_{15}$cycloalkylene, p-xylylene or hexahydroxylylene, and R, $R^2$ and $R^3$ are as defined in claim 1.

11. A process for the preparation of a compound of the formula I according to claim 1 by reacting a compound of the formula II

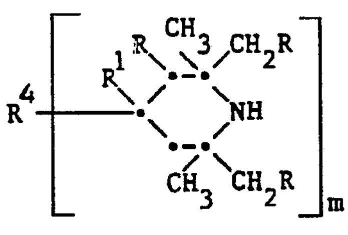

II

with phosgene in an inert solvent in the presence of a molar amount of a base, and subsequently reacting the product with a secondary amine of the formula $R^2$-NH-$R^3$, also in the presence of a molar amount of a base, it being possible to use as the base in the first stage an excess of the compound II and in the second stage also an excess of the

compound $R^2$-NH-$R^3$.

12. A process according to claim 11, according to which 0.9 - 1.2 mol of phosgene, at least one mol of the secondary amine and at least 2 mol of a base are used per mol of II.

13. A process according to claim 11, wherein a tertiary amine is used as the base.

14. A process according to claim 11, wherein the entire reaction is carried out at a temperature below 40°C.

15. A process according to claim 11 for the preparation of a compound of the formula I in which R is hydrogen.

16. A process according to claim 11 for the preparation of a compound of the formula I in which R' is hydrogen.

17. Use of a compound of claim 1 as a stabilizer for organic materials, especially for organic polymers.

18. An organic polymer containing, as a stabilizer, 0.01 to 5 % by weight of a compound of claim 1.

**Revendications**

1. Composés répondant à la formule I:

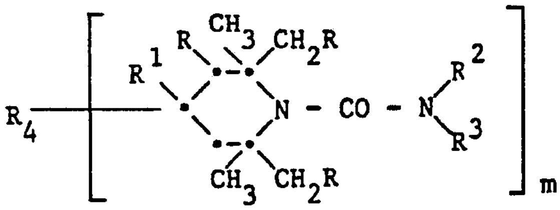

I

dans laquelle:

m désigne un nombre entier de 1 à 4,
R représente l'hydrogène ou un alkyle en $C_1$-$C_4$,
$R^1$ représente l'hydrogène, un alcoxy en $C_2$-$C_{12}$, un alcanoyloxy en $C_2$-$C_{20}$, un benzyloxy, un carbamoyloxy en $C_3$-$C_{25}$ ou -CN,
$R^2$ représente un alkyle en $C_1$-$C_{18}$, un alcoxyalkyle en $C_3$-$C_{12}$, un hydroxyalkyle en $C_2$-$C_8$, un alcényle en $C_3$-$C_{12}$, un aralkyle en $C_7$-$C_{14}$, un aryle en $C_6$-$C_{14}$, un alkylaryle en $C_7$-$C_{14}$, un cycloalkyle en $C_3$-$C_7$ ou un tétraméthyl-2,2,6,6 pipéridyle-4,
$R^3$ a l'une des significations qui ont été données pour $R^2$ ou
$R^2$ et $R^3$ forment ensemble, et avec l'atome d'azote auquel ils sont liés, un radical hétérocyclique renfermant de 5 à 7 maillons,
$R^4$ représente l'hydrogène ou un radical organique de valence égale à m, ou
$R^1$ et $R^4$ représentent ensemble un oxygène oxo ou un radical organique bivalent.

2. Composés selon la revendication 1 qui répondent à la formule III,

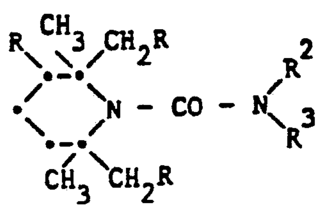

III

dans laquelle R, $R^2$ et $R^3$ ont les significations qui leur ont été données à la revendication 1.

3. Composés selon la revendication 1 qui répondent à la formule IV,

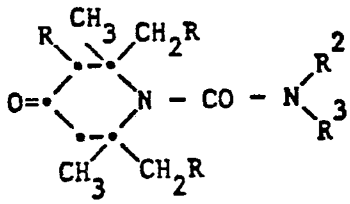

IV

dans laquelle R, $R^2$ et $R^3$ ont les significations qui leur ont été données à la revendication 1.

4. Composés selon la revendication 1 qui répondent à la formule V

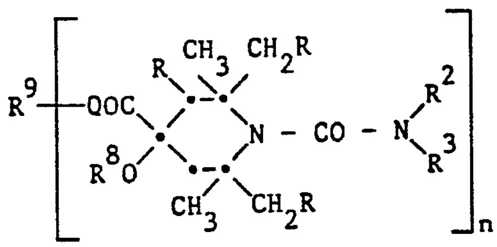

V

dans laquelle n désigne un nombre de 1 à 4, $R^8$ représente un alkyle en $C_1$-$C_4$ ou un alcanoyle en $C_2$-$C_{12}$, $R^9$ représente le radical de valence n d'un alcool en $C_1$-$C_{20}$, d'un diol en $C_2$-$C_{16}$, d'un triol en $C_3$-$C_{18}$ ou d'un tétrol en $C_4$-$C_{20}$, qui peut être interrompu par un ou plusieurs atomes d'oxygène, et R, $R^2$ et $R^3$ ont les significations qui leur ont été données à la revendication 1.

5. Composés selon la revendication 1 qui répondent à la formule VI

VI

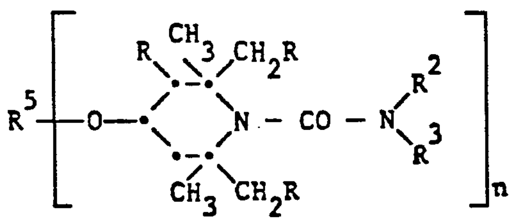

dans laquelle:

n représente un nombre entier de 1 à 4,
$R^5$ représente:
- dans le cas où n est égal à 1, l'hydrogène,
- dans le cas où n est égal à 2, un alkylène en $C_2$-$C_{12}$, un alcénylène en $C_4$-$C_{12}$, un xylylène, un radical acyle bivalent provenant d'un acide dicarboxylique aliphatique ou cycloaliphatique, d'un acide dicarbamique ou d'un acide contenant du phosphore,
- dans le cas où n est égal à 3, un radical acyle trivalent provenant d'un acide tricarboxylique aliphatique, cycloaliphatique, araliphatique, aromatique ou hétérocyclique, d'un acide tricarbamique ou d'un acide contenant du phosphore, et
- dans le cas où n est égal à 4, un radical acyle quadrivalent provenant d'un acide tétracarboxylique aliphatique, cycloaliphatique ou aromatique, et
R, $R^2$ et $R^3$ ont les significations qui leur ont été données à la revendication 1.

6. Composés selon la revendication 1 qui répondent à la formule VII

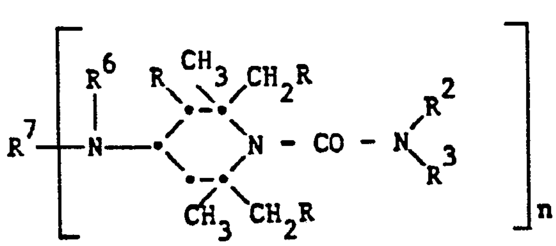

VII

dans laquelle:

n est égal à 1 ou à 2,
$R^6$ représente un alkyle en $C_1$-$C_{12}$, un alcoxy-alkyle en $C_3$-$C_{12}$, un cycloalkyle en $C_5$-$C_7$, un aralkyle en $C_7$-$C_9$, un alcanoyle en $C_2$-$C_{18}$, un alcénoyle en $C_3$-$C_5$, un benzoyle, un alcoxycarbonyle en $C_2$-$C_{13}$ ou un aryloxycarbonyle en $C_7$-$C_{11}$, et
$R^7$ représente:
- dans le cas où n est égal à 1, l'hydrogène, un alkyle en $C_1$-$C_{12}$, un alcoxyalkyle en $C_3$-$C_{12}$, un cycloalkyle en $C_5$-$C_8$, un alcényle en $C_3$-$C_8$, un glycidyle ou un cyanéthyle, et
- dans le cas où n est égal à 2, un alkylène en $C_2$-$C_{12}$, un arylène en $C_6$-$C_{15}$, un xylylène, un radical $-CH_2-CH(OH)-CH_2-$ ou un radical $-CH_2-CH(OH)-CH_2-O-D-O-CH_2-CH(OH)-CH_2-$ dans lequel D représente un alkylène en $C_2$-$C_{10}$, un arylène en $C_6$-$C_{15}$ ou un cycloalkylène en $C_6$-$C_{12}$, ou lorsque $R^6$ représente un alkyle, un cycloalkyle ou un aralkyle, $R^7$ peut aussi représenter un radical acyle bivalent provenant d'un acide dicarboxylique aliphatique, cycloaliphatique ou aromatique ou d'un acide dicarbamique,

ou

$R^6$ et $R^7$ forment ensemble et avec l'atome d'azote, dans le cadre où n est égal à 1, un radical d'imide d'un acide dicarboxylique-1,2 aliphatique, cycloaliphatique ou aromatique contenant de 4 à 12 atomes de carbone.

7. Composés selon la revendication 1 qui répondent à la formule VIII:

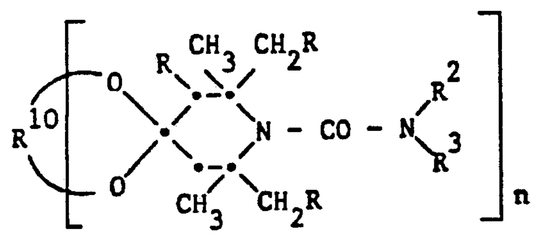

VIII

dans laquelle:

n est égal à 1 ou à 2,
$R^{10}$ représente un alkylène ou hydroxy-alkylène en $C_2$-$C_8$ ou un acyloxy-alkylène en $C_4$-$C_{22}$ lorsque n est égal à 1 ou représente le radical $(-CH_2)_2C(CH_2-)_2$ lorsque n est égal à 2, et

R, $R^2$ et $R^3$ ont les significations qui leur ont été données à la revendication 1.

8. Composés selon la revendication 1 qui répondent à la formule IX:

IX

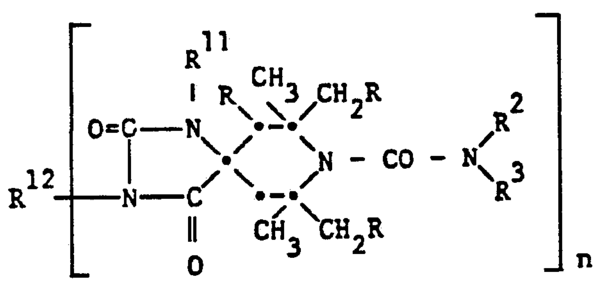

dans laquelle n est égal à 1 ou à 2, $R^{11}$ représente l'hydrogène, un alkyle en $C_1$-$C_{12}$, un allyle, un benzyle, un glycidyle ou un alcoxy-alkyle en $C_2$-$C_6$, $R^{12}$ représente, lorsque n est égal à 1, l'hydrogène, un alkyle en $C_1$-$C_{12}$, un alcényle en $C_3$-$C_5$, un aralkyle en $C_7$-$C_9$, un cycloalkyle en $C_5$-$C_8$, un hydroxyalkyle en $C_2$-$C_4$, un alcoxy-alkyle en $C_3$-$C_6$, un aryle en $C_6$-$C_{10}$ ou un glycidyle et, lorsque n est égal à 2, un alkylène en $C_2$-$C_{12}$, un arylène en $C_6$-$C_{15}$, un alcénylène en $C_4$-$C_8$ ou un radical $-CH_2-CH(OH)-CH_2-O-D-O-CH_2-CH(OH)-CH_2$ dans lequel D représente un alkylène en $C_2$-$C_{12}$, un

arylène en $C_6$-$C_{15}$ ou un cycloalkylène en $C_6$-$C_{12}$, et R, $R^2$ et $R^3$ ont les significations qui leur ont été données à la revendication 1.

9. Composés selon la revendication 1 qui répondent à la formule XII

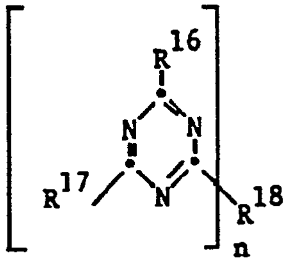

XII

dans laquelle:

n est égal à 1 ou à 2,
$R^{16}$ représente un radical de formule:

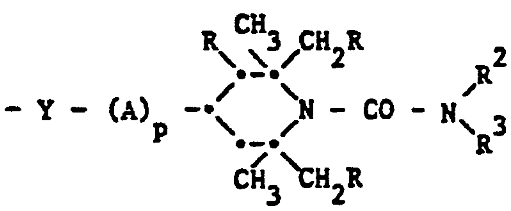

dans lequel:
R, $R^2$ et $R^3$ ont les significations qui leur ont été données à la revendication 1, Y représente -O- ou $NR^{19}$-, $R^{19}$ représente H, un alkyle en $C_1$-$C_{12}$, un hydroxyalkyle en $C_2$-$C_4$, un alcoxy-alkyle en $C_3$-$C_6$, un cyclohexyle, un benzyle ou un radical:

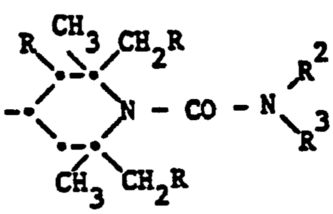

A représente un alkylène en $C_2$-$C_6$ ou $-(CH_2)_3$-O-, et p est égal à 0 ou à 1,
$R^{17}$ a une des significations qui ont été données pour $R^{16}$ ou représente un radical $-NR^{20}R^{21}$. $-OR^{22}$, $-NHC_2OR^{23}$ ou $-N(CH_2OR^{23})_2$, où $R^{20}$ a une des significations données pour $R^{19}$, $R^{21}$ représente un alkyle en $C_1$-$C_{12}$, un cyclohexyle ou un benzyle, ou $R^{20}$ et $R^{21}$ forment ensemble un alkylène en $C_4$ ou $C_5$ ou un oxa-alkylène, $R^{22}$ représente l'hydrogène, un alkyle en $C_1$-$C_{12}$ ou un phényle, et $R^{23}$ l'hydrogène ou un alkyle en $C_1$-$C_4$, et
$R^{18}$, lorsque n est égal à 1, a l'une des significations qui ont été données pour $R^{16}$ et $R^{17}$ et, lorsque n est égal à 2, représente un radical -Y-Q-Y- dans lequel Q représente un alkylène en $C_4$-$C_{12}$, un alkylène en $C_2$-$C_{12}$ interrompu par -O-, par -NH-, par -N(alkyl)- ou par un radical de formule:

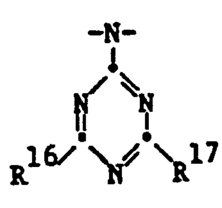

un cyclohéxyle, un xylène, ou phénylène.

10. Composés selon la revendication 1 qui répondent à la formule XIII

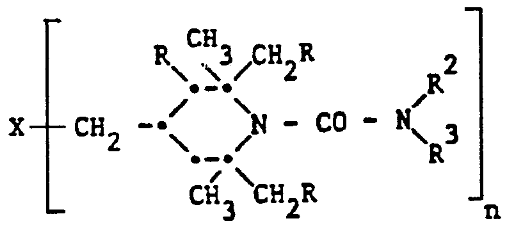

XIII

dans laquelle:

n est égal à 1 ou 2,
X représente:

- lorsque n est égal à 1, un radical -CN, $-COOR^{24}$, $-CH_2NH_2$, $-CH_2OH$ ou $-CH_2COOR^{24}$, le symbole $R^{24}$ désignant un alkyle en $C_1$-$C_{18}$, un benzyle ou un cycloalkyle, et
- lorsque n est égal à 2, un radical $-COO-R^{25}-OOC$ dans lequel $R^{25}$ représente un alkylène en $C_2$-$C_{12}$, un alkylène en $C_4$-$C_{10}$ interrompu par -O- ou par un radical -N(alkyl)- à alkyle en $C_1$-$C_4$, un cycloalkylène en $C_6$-$C_{15}$, un p-xylylène ou un hexahydro-xylylène, et

R, $R^2$ et $R^3$ ont des significations qui leur ont été données à la revendication 1.

11. Procédé pour préparer des composés de formule I selon la revendication 1, procédé selon lequel on fait réagir des composés de formule II

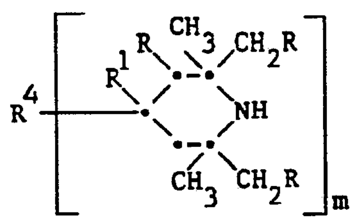

II

avec le phosgène dans un solvant inerte, en présence de quantités molaires d'une base, puis on fait réagir avec une amine secondaire de formule $R^2$-NH-$R^3$, également en présence de quantités molaires d'une base, et, dans la première étape, on peut aussi utiliser, comme base, un excès du composé (II) et, dans la seconde étape, également un excès du composé $R^2$-NH-$R^3$.

12. Procédé selon la revendication 11 selon lequel on utilise, par mole du composé (II), de 0,9 à 1,2 mol de phosgène, au moins 1 mol de l'amine secondaire et au moins 2 mol d'une base.

13. Procédé selon la revendication 11 caractérisé en ce qu'on utilise comme base une amine tertiaire.

14. Procédé selon la revendication 11 caractérisé en ce qu'on effectue l'ensemble de la réaction à des températures inférieures à 40°C.

15. Procédé selon la revendication 11 appliqué à la préparation de composés de formule I dans lesquels R représente l'hydrogène.

16. Procédé selon la revendication 11 appliqué à la préparation de composés de formule I dans lesquels R' représente l'hydrogène.

17. Application des composés selon la revendication 1 comme stabilisants pour des matières organiques, plus spécialement pour des polymères organiques.

18. Polymère organique contenant, comme stabilisant, de 0,01 à 5 % en poids d'un composé selon la revendication 1.